# EUROPEAN PATENT SPECIFICATION

(11) **EP 3 131 577 B1**
(45) Date of publication and mention of the grant of the patent: **22.04.2020**
(21) Application number: 14784210.8
(22) Date of filing: 13.10.2014
(51) Int. Cl.: C12N 15/52, C12N 15/70, C12N 1/21, C07K 14/21, A61K 39/104

(54) **MODIFIED HOST CELLS AND USES THEREOF**
MODIFIZIERTE WIRTSZELLEN UND VERWENDUNGEN DAVON
CELLULES HÔTES MODIFIÉES ET LEURS UTILISATIONS

(30) Priority: 17.04.2014 US 201461980988 P
(43) Date of publication of application: 22.02.2017
(73) Proprietor: GlaxoSmithKline Biologicals S.A., 1330 Rixensart (BE)
(72) Inventor: KOWARIK, Michael, 8055 Zurich (CH); KEMMLER, Stefan J., 8047 Zurich (CH); QUÉBATTE, Julien L., 8952 Schlieren (CH); FERON, Christiane Marie-Paule Simone Jeanne, 1330 Rixensart (BE)
(74) Representative: Johnston, Caroline Louise
(86) International application number: PCT/EP2014/071898
(87) International publication number: WO 2015/158403

(56) References cited:
- WO-A1-2011/062615
- WO-A1-2011/138361
- WO-A1-2013/034664
- WO-A1-2014/037585
- WO-A1-2014/057109
- WO-A1-2014/072405
- WO-A2-2006/119987
- WO-A2-2009/104074
- IHSSEN JULIAN ET AL: "Production of glycoprotein vaccines in Escherichia coli", MICROBIAL CELL FACTORIES, BIOMED CENTRAL, LONDON, NL, vol. 9, no. 1, 11 August 2010 (2010-08-11) , page 61, XP021077209, ISSN: 1475-2859, DOI: 10.1186/1475-2859-9-61
- RAYMOND CHRISTOPHER K ET AL: "Genetic variation at the O-antigen biosynthetic locus in Pseudomonas aeruginosa", JOURNAL OF BACTERIOLOGY, AMERICAN SOCIETY FOR MICROBIOLOGY, US, vol. 184, no. 13, 1 July 2002 (2002-07-01) , pages 3614-3622, XP002593462, ISSN: 0021-9193, DOI: 10.1128/JB.184.13.3614-3622.2002
- Myriam Belanger ET AL: "Functional analysis of genes responsible for the synthesis of the B-band O antigen of Pseudomonas aeruginosa serotype O6 lipopolysaccharide", Microbiology, 1 December 1999 (1999-12-01), pages 3505-3521, XP055158764, ENGLAND Retrieved from the Internet: URL:http://mic.sgmjournals.org/cgi/content /abstract/145/12/3505
- C. R. Dean ET AL: "Characterization of the Serogroup O11 O-Antigen Locus of Pseudomonas aeruginosa PA103", Journal of Bacteriology, 15 July 1999 (1999-07-15), pages 4275-4284, XP055158523, UNITED STATES Retrieved from the Internet: URL:http://jb.asm.org/content/181/14/4275. abstract
- KNIREL YURIY A ET AL: "Conserved and variable structural features in the lipopolysaccharide of Pseudomonas aeruginosa", JOURNAL OF ENDOTOXIN RESEARCH, vol. 12, no. 6, 2006, pages 324-336, XP009181742, ISSN: 0968-0519
- WACKER MICHAEL ET AL: "Prevention of Staphylococcus aureus Infections by Glycoprotein Vaccines Synthesized in Escherichia coli", JOURNAL OF INFECTIOUS DISEASES, vol. 209, no. 10, May 2014 (2014-05), pages 1551-1561, XP009181722,
- MARIO F FELDMAN ET AL: "Engineering N-linked protein glycosylation with diverse O antigen lipopolysaccharide structures in Escherichia coli", PROCEEDINGS OF THE NATIONAL ACADEMY OF SCIENCES, NATIONAL ACADEMY OF SCIENCES, US, vol. 102, no. 8, 22 February 2005 (2005-02-22), pages 3016-3021, XP009121034, ISSN: 0027-8424, DOI: 10.1073/PNAS.0500044102 [retrieved on 2005-02-09]
- MUNIR AL-ZEER ET AL: "LPS-based conjugate vaccines composed of O-polysaccharide from Pseudomonas aeruginosa IATS 6 and 11 bound to a carrier protein", WORLD JOURNAL OF MICROBIOLOGY AND BIOTECHNOLOGY, KLUWER ACADEMIC PUBLISHERS, DO, vol. 23, no. 11, 13 May 2007 (2007-05-13), pages 1541-1549, XP019557904, ISSN: 1573-0972, DOI: 10.1007/S11274-007-9399-2
- COIN D ET AL: "A Pseudomonas aeruginosa alginate-exotoxin A conjugate that elicits anti-alginate and exotoxin A-neutralizing antibodies", FEMS MICROBIOLOGY LETTERS, WILEY-BLACKWELL PUBLISHING LTD, GB, vol. 76, no. 4, 1 August 1991 (1991-08-01) , pages 185-192, XP023916908, ISSN: 0378-1097, DOI: 10.1111/J.1574-6968.1991.TB04213.X [retrieved on 1991-08-01]

### SEQUENCE LISTING

<110> GlaxoSmithKline Biologicals S.A.
   GlycoVaxyn AG
<120> MODIFIED HOST CELLS AND USES THEREOF
<130> 103651PC
<140> TBA
   <141> On even date herewith
<150> 61/980,988
   <151> 2014-04-17
<160> 3
<170> PatentIn version 3.5
<210> 1
   <211> 5
   <212> PRT
   <213> Artificial Sequence
<220>
   <223> N-Glycosylation Consensus Sequence
<220>
   <221> MISC_FEATURE
   <222> (1)..(1)
   <223> Xaa = Asp or Glu
<220>
   <221> MISC_FEATURE
   <222> (2)..(2)
   <223> Xaa = any natural amino acid except Pro
<220>
   <221> MISC_FEATURE
   <222> (4)..(4)
   <223> Xaa = any natural amino acid except Pro
<220>
   <221> MISC_FEATURE
   <222> (5)..(5)
   <223> Xaa = Ser or Thr
<400> 1
   Xaa Xaa Asn Xaa Xaa
   1               5
<210> 2
   <211> 558
   <212> PRT
   <213> Pseudomonas aeruginosa O6
<220>
   <223> formyltransferase
<400> 2
   Met Ser Trp Gln Leu Phe Ser Glu Lys Cys Arg Phe Leu Gly Ala Val
   1               5                   10                  15
   Glu Ile Ser Gln His Phe Trp Gly Phe Ile Val Leu Glu Ala Ser Phe
               20                  25                  30
   Gly Met Lys Ile Lys Ala Ala Leu Ile Val Asp Asp Leu Ser Leu Ser
           35                  40                  45
   Glu Trp Gln Lys Arg Ala Ile Glu Asp Ser Ser Glu Tyr Leu Asp Ile
       50                  55                  60
   Gln Leu Val Leu Ser Cys Arg Asn Ser Ala Thr Lys Lys Ser Val Ile
   65                  70                  75                  80
   Lys His Cys Gly Tyr Tyr Phe Leu Asn Ile Leu Ser Leu Lys Asn Asp
                   85                  90                  95
   Met Thr Arg Arg Val Gln Leu Asp Ser Arg Gly Ser Glu Val Ile His
               100                 105                 110
   Phe Asp Ser Asp Tyr Glu Gly Ala Trp Gln Arg Ile Pro Glu Asp Val
           115                 120                 125
   Cys Ala Arg Ile Leu Asp Lys Gly Ile Lys Leu Val Ile Lys Phe Gly
       130                 135                 140
   Met Ser Leu Leu Arg Ile Asp Gly Gly Leu Gln Arg Leu Asp Ile Leu
   145                 150                 155                 160
   Ser Tyr His His Gly Asp Pro Glu Tyr Tyr Arg Gly Arg Pro Ala Gly
                   165                 170                 175
   Phe Tyr Glu Ile Tyr Glu Asn Ala Asp Ser Val Gly Ile Ile Val Gln
               180                 185                 190
   Lys Leu Ser Asn Lys Leu Asp Ala Gly Glu Val Leu Val Arg Gly Tyr
           195                 200                 205
   Ser Lys Val His His His Ser Tyr Lys Lys Thr Ser Arg Asn Phe Tyr
       210                 215                 220
   Leu Asn Ser Val Val Leu Leu Arg Lys Ala Leu Val Asn Tyr Ser Arg
   225                 230                 235                 240
   Gly Glu Gln Val Val Leu Glu Lys Leu Gly Lys Asn Tyr Arg Leu Pro
                   245                 250                 255
   Ser Asn Phe Thr Val Phe Lys Phe Phe Cys Lys Thr Ile Phe Arg Gly
               260                 265                 270
   Leu Ala Arg Leu Ser Tyr Gly Ala Phe Phe Glu Lys Lys Trp Asn Val
           275                 280                 285
   Val Ala Leu Pro Tyr Asn Asp Ile Pro Ser Leu Gln Glu Leu Ser Val
       290                 295                 300
   Ser Ala Gly Lys Ile Pro Lys Val Glu Lys Gly Tyr Thr Phe Tyr Ala
   305                 310                 315                 320
   Asp Pro Phe Phe Ser Ala Asp Gly Lys Leu Ile Arg Leu Glu Ala Leu
                   325                 330                 335
   Asn Ala Ser Asn Gly Leu Gly Glu Ile Ile Glu Leu Lys Ala Gln Ser
               340                 345                 350
   Leu Asp Phe Ser Arg Val Ile Leu Lys Gly Asn His Phe Ser Tyr Pro
           355                 360                 365
   Tyr Ser Phe Glu Ala Ser Gly Val Glu Tyr Leu Ile Pro Glu Val Ala
       370                 375                 380
   Ser His Ser Ala Pro Cys Leu Leu Pro Pro Pro Phe Ala Leu Glu Ser
   385                 390                 395                 400
   Lys Lys Leu Phe Gln Gly Met Glu Gly Glu Arg Ile Leu Asp Gly Thr
                   405                 410                 415
   Leu Phe Glu His Gly Gly Arg Tyr Tyr Leu Phe Cys Gly Gln Ala Val
               420                 425                 430
   Ser Gly Ser Asp Asn Leu Tyr Leu Tyr Val Gly Glu Ser Leu Glu Gly
           435                 440                 445
   Pro Tyr Thr Ser His Pro Cys Asn Pro Val Val Met Asn Pro Gly Ser
       450                 455                 460
   Ala Arg Met Gly Gly Arg Ile Phe Lys Glu Gly Gly Lys Leu Tyr Arg
   465                 470                 475                 480
   Phe Gly Gln Asn Asn Ser Tyr Gly Tyr Gly Ser Ser Leu Ala Val Asn
                   485                 490                 495
   Glu Ile Glu Val Leu Asp Pro Glu His Tyr Ser Glu Lys Arg Val Ala
               500                 505                 510
   Asn Leu Ala Phe Gln Asp Ala Arg Gly Pro His Thr Ile Asp Ile His
   515 520 525
   Gly Gln Thr Met Ile Leu Asp Phe Tyr Gln Asp Arg Phe Ser Leu Leu
   530 535 540
   Ala Gly Tyr Arg Arg Leu Val Ala Arg Leu Leu Ser Arg Gly
   545 550 555
<210> 3
   <211> 417
   <212> PRT
   <213> Pseudomonas aeruginosa O6
<220>
   <223> wzy polymerase
<400> 3
   Met Tyr Ala Met Leu Thr Gly Ala Thr Leu Leu Ile Phe Ala Val Ala
   1 5 10 15
   Ala Arg Leu Leu Ala Arg Ser Ala Ile His Pro Ser Val Ala Met Pro
   20 25 30
   Ile Thr Trp Gly Leu Gly Leu Ile Gly Val Ser Leu Ala Ser Leu Ile
   35 40 45
   Gly Phe Tyr Arg Val Glu Ser Asp Ala Leu Leu Ile Phe Leu Phe Gly
   50 55 60
   Val Met Ser Phe Ser Leu Ser Ala Gly Cys Phe Ser Phe Leu Tyr Asn
   65 70 75 80
   Gly Tyr Phe Arg Ala Pro Ser Ser Asn Phe Leu Phe Asp Ser Glu Leu
   85 90 95
   Arg Thr Arg Ala Leu Val Ile Phe Phe Cys Leu Ala His Ile Val Phe
   100 105 110
   Leu Thr Val Ile Tyr Arg Asp Leu Ser Ser Ile Ala Pro Thr Leu Arg
   115 120 125
   Glu Ala Ala Tyr Met Ala Arg Ala Gln Ser Val Ser Gly Glu Pro Val
   130 135 140
   Leu Ser Ser Leu Ser Met Asn Tyr Leu Gln Leu Gly Gln Thr Val Ile
   145 150 155 160
   Pro Leu Val Val Leu Leu Tyr Leu Arg Gly Lys Cys Gly Val Leu Gly
   165 170 175
   Phe Leu Ala Ile Ser Val Pro Trp Met Gly Val Ile Leu Leu Ala Ser
   180 185 190
   Gly Arg Ala Ser Leu Met Gln Met Leu Val Gly Leu Phe Phe Ile Tyr
   195 200 205
   Ile Leu Val Lys Gly Ser Pro Ser Leu Lys Ser Leu Leu Val Ile Gly
   210 215 220
   Leu Ala Met Phe Leu Val Ile Ala Val Gly Ala Val Ala Thr Ser Lys
   225 230 235 240
   Ile Gln Phe His Glu Gly Asp Gly Ile Ser Thr Leu Phe Ile Glu Leu
   245 250 255
   Tyr Arg His Val Ala Gly Tyr Ala Leu Gln Gly Pro Val Leu Phe Asp
   260 265 270
   Arg Tyr Tyr Gln Gly Ser Ile His Leu Glu Pro Tyr Trp Ser Pro Leu
   275 280 285
   Asn Gly Phe Cys Ser Ile Leu Ala Thr Val Gly Leu Cys Gln Lys Pro
   290 295 300
   Pro Leu His Leu Asp Phe Tyr Glu Tyr Ala Pro Gly Glu Leu Gly Asn
   305 310 315 320
   Val Tyr Ser Met Phe Phe Ser Met Tyr Pro His Tyr Gly Ala Leu Gly
   325 330 335
   Val Ile Gly Val Met Ala Leu Tyr Gly Met Leu Cys Ser Tyr Ala Tyr
   340 345 350
   Cys Lys Ala Lys Lys Gly Ser Leu Tyr Phe Thr Val Leu Ser Ser Tyr
   355 360 365
   Leu Phe Ser Ala Ile Val Phe Ser Leu Phe Ser Asp Gln Ile Ser Thr
   370 375 380
   Ser Trp Trp Phe Tyr Val Lys Met Thr Ile Ile Leu Gly Ile Leu Cys
   385 390 395 400
   Phe Val Phe Arg Arg Asp Arg Met Phe Val Ile Arg Leu Pro Gln Ala
   405 410 415
   Gly

## Description

### 1. INTRODUCTION

Described herein are modified host cells useful in the production of bioconjugates that can be used to vaccinate subjects against infection with *Pseudomonas.* The genomes of the modified host cells described herein comprise genes that encode proteins involved in glycosylation of proteins as well as genes specific to the production of *Pseudomonas*-specific antigens.

### 2. BACKGROUND

Disease caused by infection with strains of *Pseudomonas* (*e.g., P. aeruginosa*) represents a major threat worldwide. While development of vaccines against such infection is ongoing, there remains a major need for effective vaccines against *Pseudomonas* infection that can safely be produced in high quantities. Relevant references include WO 09/104074, WO 06/119987, WO 11/062615, WO 13/034664, WO 14/037585, WO 11/138361, Ihssen et al Production of glycoprotein vaccines in Escherichia coli Microbial Cell Factories Vol 9 page 61 (2010), Raymond et al J. Bacteriology 184, 3614-3622 (2002), Belanger et al Microbiology, 1 December 1999, pages 3505-3521, Dean et al J. Bacteriology 15th July 1999 page 4275-4284, Knirel Yuriy A et al J. Endotoxin Research vol 12, pages 324-336 (2006), Wacker M et al J. Infect. Dis. 209; 1551-1561 (2014), WO 14/072405 and WO 14/057109, Munir Al-Zeer et al World Journal of Microbiology and Biotechnology 23; 1541-1549 (2007) and Coin D et al FEMS Microbiology Letters 76; 185-192 (1991).

### 3. SUMMARY

The invention provides a host cell comprising:
(i) a nucleic acid that encodes a glycosyltransferase derived from an *rfb* cluster of
   *Pseudomonas;*
(ii) a nucleic acid that encodes a formyltransferase enzyme, wherein said nucleic acid encodes a protein having about or at least 80%, 85%, 90%, 95%, 96%, 97%, 98%, or 99% identity or homology to SEQ ID NO:2, or wherein said nucleic acid encodes SEQ ID NO:2;
(ii) a nucleic acid that encodes an oligosaccharyl transferase; and
(iii) a nucleic acid that encodes a carrier protein comprising an N-glycosylation consensus sequence D/E - X - N - X- S/T, wherein X is any amino acid except proline.

In one aspect, provided herein is a modified prokaryotic host cell comprising nucleic acids encoding enzymes capable of producing a bioconjugate comprising a *Pseudomonas* antigen. Such host cells may naturally express nucleic acids specific for production of a *Pseudomonas* antigen, or the host cells may be made to express such nucleic acids, *i.e.,* in certain embodiments said nucleic acids are heterologous to the host cells. In certain embodiments, one or more of said nucleic acids specific for production of a *Pseudomonas* antigen are heterologous to the host cell and intergrated into the genome of the host cell. In certain embodiments, the host cells provided herein comprise nucleic acids encoding additional enzymes active in the N-glycosylation of proteins, e.g., the host cells provided herein further comprise a nucleic acid encoding an oligosaccharyl transferase and/or one or more nucleic acids encoding other glycosyltransferases. In certain embodiments, the host cells provided herein comprise a nucleic acid encoding a carrier protein, *e.g.,* a protein to which oligosaccharides and/or polysaccharides can be attached to form a bioconjugate. In a specific embodiment, the host cell is *E. coli.*

In a specific embodiment, provided herein is a modified prokaryotic host cell comprising nucleic acids encoding enzymes capable of producing a bioconjugate comprising a *Pseudomonas* antigen, wherein said host cell comprises an *rfb* cluster from *Pseudomonas* or a glycosyltransferase derived from an *rfb* cluster from *Pseudomonas.* In a specific embodiment, said *rfb* cluster from *Pseudomonas* or glycosyltransferase derived from an *rfb* cluster from *Pseudomonas* is integrated into the genome of said host cell. In another specific embodiment, said *rfb* cluster from *Pseudomonas* or glycosyltransferase derived from an *rfb* cluster from *Pseudomonas* is an *rfb* cluster or glycosyltransferase from *Pseudomonas aeruginosa.* See Raymond et al., J Bacteriol., 2002 184(13):3614-22. In a specific embodiment, said *rfb* cluster from *Pseudomonas aeruginosa* is the *rfb* cluster from any one of the serotypes O1-O20. In another specific embodiment, said *rfb* cluster from *Pseudomonas aeruginosa* is the *rfb* cluster from any one of the serotypes described in Knirel et al., 2006, Journal of Endotoxin Research 12(6): 324-336. In a specific embodiment, said *rfb* cluster from *Pseudomonas aeruginosa* is the *rfb* cluster from serotype O2, O5, O6, O11, 015, 016. In a specific embodiment, said *rfb* cluster from *Pseudomonas aeruginosa* is the *rfb* cluster from serotype O6. In a specific embodiment, said *rfb* cluster from *Pseudomonas aeruginosa* is the *rfb* cluster from serotype O11. In another specific embodiment, said host cell comprises a nucleic acid encoding an oligosaccharyl transferase (*e.g., pglB* from *Campylobacter jejuni*). In another specific embodiment, said nucleic acid encoding an oligosaccharyl transferase (*e.g., pglb* from *Campylobacter jejuni*) is integrated into the genome of the host cell. In a specific embodiment, said host cell comprises a nucleic acid encoding a carrier protein. In another specific embodiment, the host cell is *E. coli.*

In certain embodiments of the disclosure, said modified prokaryotic host cell comprising nucleic acids encoding enzymes capable of producing a bioconjugate comprising a *Pseudomonas* antigen, further comprises one or more accessory enzymes, including branching, modifying, amidiating, chain length regulating, acetylating, formylating, polymerizing enzymes. In a specific embodiment, said one or more accessory enzymes are heterologous to the host cell. In a specific embodiment, said one or more accessory enzymes are inserted into the genome of the modified prokaryotic host cell in addition to the *rfb* cluster. In a specific embodiment, said one or more accessory enzymes are derived from *Pseudomonas,* e.g., *P. aeruginosa.*

In a specific embodiment of the disclosure, a modified prokaryotic host cell provided herein comprises a nucleic acid that encodes a formyltransferase. In another specific embodiment, said formyltransferase is the formyltransferase presented in SEQ ID NO:2, or a homolog thereof. In another specific embodiment, said formyltransferase is incorporated (e.g., inserted into the genome of or plasmid expressed by) in said host cell as part of a *Pseudomonas rfb* cluster, wherein said *Pseudomonas rfb* cluster has been modified to comprise the formyltransferase. In another specific embodiment, said *Pseudomonas rfb* cluster is a *Pseudomonas aeruginosa* serotype O6 *rfb* cluster.

In another specific embodiment of the disclosure, a modified prokaryotic host cell provided herein comprises a nucleic acid that encodes a wzy polymerase. In another specific embodiment, said wzy polymerase is the wzy polymerase presented in SEQ ID NO:3, or a homolog thereof. In another specific embodiment, said wzy polymerase is incorporated (e.g., inserted into the genome of or plasmid expressed by) in said host cell as part of a *Pseudomonas rfb* cluster, wherein said *Pseudomonas rfb* cluster has been modified to comprise the wzy polymerase. In another specific embodiment, said *Pseudomonas rfb* cluster is a *Pseudomonas aeruginosa* serotype O6 *rfb* cluster.

In another specific embodiment of the disclosure, a modified prokaryotic host cell provided herein comprises (i) a nucleic acid that encodes a formyltransferase and (ii) a nucleic acid that encodes a wzy polymerase. In a specific embodiment, said formyltransferase is the formyltransferase presented in SEQ ID NO:2, or a homolog thereof. In another specific embodiment, said wzy polymerase is the wzy polymerase presented in SEQ ID NO:3, or a homolog thereof. In a specific embodiment, said formyltransferase and said wzy polymerase are incorporated (e.g., inserted into the genome of or plasmid expressed by) in said host cell as part of a *Pseudomonas rfb* cluster, wherein said *Pseudomonas rfb* cluster has been modified to comprise the formyltransferase and wzy polymerase. In another specific embodiment, said *Pseudomonas rfb* cluster is a *Pseudomonas aeruginosa* serotype O6 *rfb* cluster.

In another aspect of the disclosure, provided herein is an isolated nucleic acid sequence encoding a modified *Pseudomonas rfb* cluster, e.g., *Pseudomonas aeruginosa* serotype O6 *rfb* cluster, wherein said modified *Pseudomonas rfb* cluster comprises (i) a gene encoding a formyltransferase (e.g., a gene encoding SEQ ID NO:2 or a homolog thereof), (ii) a gene encoding a *wzy* polymerase (e.g., a gene encoding SEQ ID NO:3 or a homolog thereof); or (iii) a gene encoding a formyltransferase (e.g., a gene encoding SEQ ID NO:2 or a homolog thereof) and a gene encoding a wzy polymerase (e.g., a gene encoding SEQ ID NO3 or a homolog thereof).

Nucleic acids that encode formyltransferases and nucleic acids that encode wzy polymerases that are use to generate modified *Pseudomonas rfb* clusters, e.g., modified *Pseudomonas aeruginosa* serotype O6 *rfb* clusters, can be inserted into the *rfb* cluster at multiple positions and in multiple orientations.

In a specific embodiment of the disclosure, the gene encoding said formyltransferase and/or the gene encoding said wzy polymerase is/are inserted downstream of the genes of the *Pseudomonas rfb* cluster, e.g., the *Pseudomonas aeruginosa* serotype O6 *rfb* cluster. In a specific emnodiment, the the gene encoding said formyltransferase and/or the gene encoding said wzy polymerase is/are inserted downstream of the *wbpM* gene of the *Pseudomonas aeruginosa* serotype O6 *rfb* cluster.

In a specific embodiment of the disclosure, the gene encoding said formyltransferase and/or the gene encoding said *wzy* polymerase is/are inserted upstream of the genes of the *Pseudomonas rfb* cluster, e.g., the *Pseudomonas aeruginosa* serotype O6 *rfb* cluster. In a specific emnodiment of the disclosure, the the gene encoding said formyltransferase and/or the gene encoding said *wzy* polymerase is/are inserted downstream of the *wzz* gene of the *Pseudomonas aeruginosa* serotype O6 *rfb* cluster.

In a specific embodiment of the disclosure, the gene encoding said formyltransferase and/or the gene encoding said wzy polymerase is/are inserted in a clockwise orientation relative to the genes of the *Pseudomonas rfb* cluster, e.g., the *Pseudomonas aeruginosa* serotype O6 *rfb* cluster.

In a specific embodiment of the disclosure, the gene encoding said formyltransferase and/or the gene encoding said wzy polymerase is/are inserted in a counter-clockwise orientation relative to the genes of the *Pseudomonas rfb* cluster, e.g., the *Pseudomonas aeruginosa* serotype O6 *rfb* cluster.

Another aspect of the invention is a method of producing a bioconjugate comprising a *Pseudomonas* antigen linked to a carrier protein, said method comprising culturing a host cell comprising:
(iii) a nucleic acid that encodes a glycosyltransferase derived from an *rfb* cluster of *Pseudomonas;*
(iv) a nucleic acid that encodes a formyltransferase enzyme, wherein said nucleic acid encodes a protein having about or at least 80%, 85%, 90%, 95%, 96%, 97%, 98%, or 99% identity or homology to SEQ ID NO:2, or wherein said nucleic acid encodes SEQ ID NO:2;
(iv) a nucleic acid that encodes an oligosaccharyl transferase; and
(v) a nucleic acid that encodes a carrier protein comprising an N-glycosylation consensus sequence D/E - X - N - X- S/T, wherein X is any amino acid except proline,
under conditions suitable for the production of proteins, and optionally purifying the N-glycosylated carrier protein. Methods for producing proteins using host cells, e.g., *E*. *coli,* and isolating proteins produced by host cells, are well-known in the art.

In another aspect, provided herein are bioconjugates produced by the host cells provided herein.

In a specific embodiment, provided herein is a bioconjugate comprising a Pseudomonas aeruginosa serotype O6 antigen conjugated to a carrier protein via the N residue of at least one N-glycosylation consensus sequence D/E-X-N-X-S/T, wherein X is any amino acid except proline, wherein the O6 antigen is formylated.

In another aspect of the disclosure, provided herein are compositions, *e.g.,* pharmaceutical compositions, comprising one or more of the bioconjugates provided herein.

In a specific embodiment of the disclosure, provided herein is a composition, *e.g.,* pharmaceutical composition, comprising a bioconjugate comprising a carrier protein linked to a *Pseudomonas* antigen. In a specific embodiment, said *Pseudomonas* antigen is an O antigen of *Pseudomonas aeruginosa.*

In another aspect of the disclosure, provided herein are methods of preventing infection of a subject, *e.g.,* a human subject, by *Pseudomonas* (*e.g., Pseudomonas aeruginosa*), comprising administering to the subject an effective amount of a composition described herein.

In another aspect of the disclosure, provided herein are methods of treating a subject, *e.g.,* a human subject, that has been infected by *Pseudomonas* (*e.g., Pseudomonas aeruginosa*), comprising administering to the subject an effective amount of a composition described herein.

In another aspect of the disclosure, provided herein are methods of inducing an immune response in a subject, *e.g.,* a human subject, against *Pseudomonas* (*e.g., Pseudomonas aeruginosa*), comprising administering to the subject an effective amount of a composition described herein.

### 3.1 Terminology

OPS: O polysaccharide; the O antigen of Gram-negative bacteria. OPS also are referred to herein as O antigen.

LPS: lipopolysaccharide.

*rfb* cluster: a gene cluster that encodes enzymatic machinery capable of synthesis of an O antigen backbone structure.

*waaL:* the O antigen ligase gene encoding a membrane bound enzyme with an active site located in the periplasm. The encoded enzyme transfers undecaprenylphosphate (UPP)-bound O antigen to the lipid A core, forming lipopolysaccharide.

RU: repeat unit. As used herein, the RU is set equal to the Biological repeat unit, BRU. The BRU describes the RU of an O antigen as it is synthesized *in vivo.*

Und-PP: undecaprenyl pyrophosphate.

LLO: lipid linked oligosaccharide.

As used herein, the term "bioconjugate" refers to conjugate between a protein (*e.g.,* a carrier protein) and an antigen, e.g., a *Pseudomonas* antigen, prepared in a host cell background, wherein host cell machinery links the antigen to the protein (*e.g*., N-links).

The term "about," when used in conjunction with a number, refers to any number within ±1, ±5 or ±10% of the referenced number.

As used herein, the term "effective amount," in the context of administering a therapy (*e.g.,* a composition described herein) to a subject refers to the amount of a therapy which has a prophylactic and/or therapeutic effect(s). In certain embodiments, an "effective amount" refers to the amount of a therapy which is sufficient to achieve one, two, three, four, or more of the following effects: (i) reduce or ameliorate the severity of a *Pseudomonas* infection or symptom associated therewith; (ii) reduce the duration of a *Pseudomonas* infection or symptom associated therewith; (iii) prevent the progression of a *Pseudomonas* infection or symptom associated therewith; (iv) cause regression of a *Pseudomonas* infection or symptom associated therewith; (v) prevent the development or onset of a *Pseudomonas* infection, or symptom associated therewith; (vi) prevent the recurrence of a *Pseudomonas* infection or symptom associated therewith; (vii) reduce organ failure associated with a *Pseudomonas* infection; (viii) reduce hospitalization of a subject having a *Pseudomonas* infection; (ix) reduce hospitalization length of a subject having a *Pseudomonas* infection; (x) increase the survival of a subject with a *Pseudomonas* infection; (xi) eliminate a *Pseudomonas* infection in a subject; (xii) inhibit or reduce a *Pseudomonas* replication in a subject; and/or (xiii) enhance or improve the prophylactic or therapeutic effect(s) of another therapy.

As used herein, the term "in combination," in the context of the administration of two or more therapies to a subject, refers to the use of more than one therapy. The use of the term "in combination" does not restrict the order in which therapies are administered to a subject. For example, a first therapy (*e.g.,* a composition described herein) can be administered prior to (*e.g.,* 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.,* 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a subject.

As used herein, the term "subject" refers to an animal (*e.g*., birds, reptiles, and mammals). In another embodiment, a subject is a mammal including a non-primate (*e.g*., a camel, donkey, zebra, cow, pig, horse, goat, sheep, cat, dog, rat, and mouse) and a primate (*e.g.,* a monkey, chimpanzee, and a human). In certain embodiments, a subject is a non-human animal. In some embodiments, a subject is a farm animal or pet (*e.g.,* a dog, cat, horse, goat, sheep, pig, donkey, or chicken). In a specific embodiment, a subject is a human. The terms "subject" and "patient" may be used herein interchangeably.

### 4. BRIEF DESCRIPTION OF THE DRAWINGS

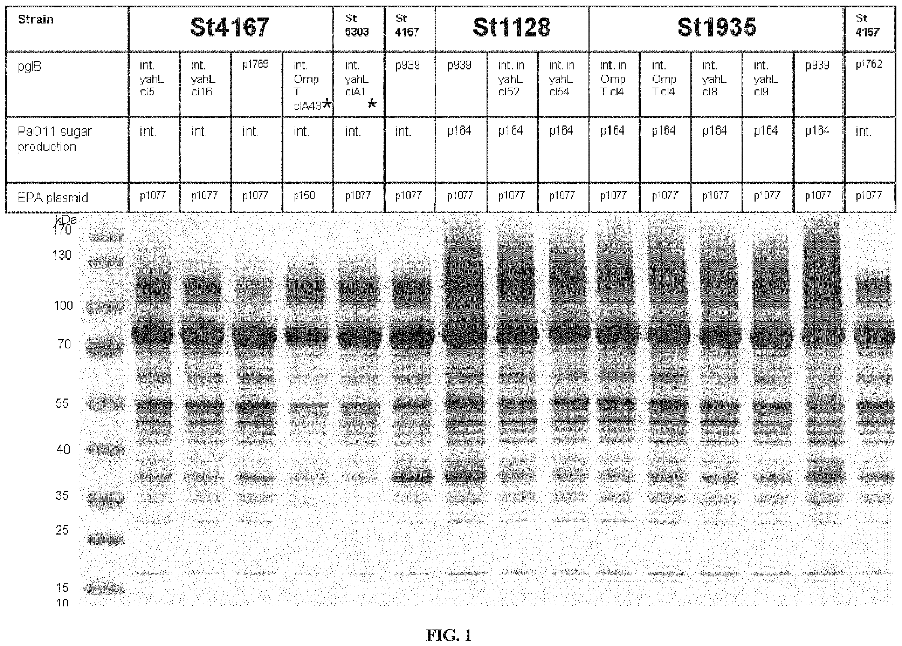

Fig. 1 depicts a Western blot of periplasmic extracts from modified host cells that produce bioconjugates. Strains as described in the Examples are indicated. "Int." refers to an integrated component. "*" refers to integration using a transposon-mediated approach.

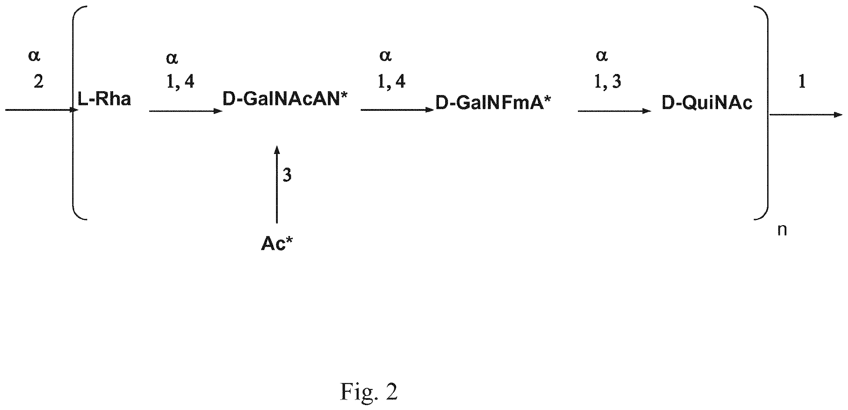

Fig. 2 depicts the repeat unit structure of the O6 O antigen of *Pseudomonas aeruginosa.* * indicates positions that can vary in their chemical composition according to subserotype identity. Variability is introduced by the activity of amidases that convert the acid functions of GalNAcA residues at C6 to amide, resulting in GalNAcAN (or GalNFmA to GalNFmAN; an acetyl group substitutes C3 of the GalNAcAN* residue in some subserotypes). The genes for polymerization of the repeat unit (wzy), acetylation, formylation, and amidation of one of the Ga1NX residues are unknown. L-Rha, L-Rhamnose; D-GalNAcAN, 6-amido-2-N-acetyl-D-galactosaminuronic acid; D-GalNFmAN, 2-N-formyl-D-galactosaminuronic; D-QuiNAc, N-acetyl-D-quinosamine.

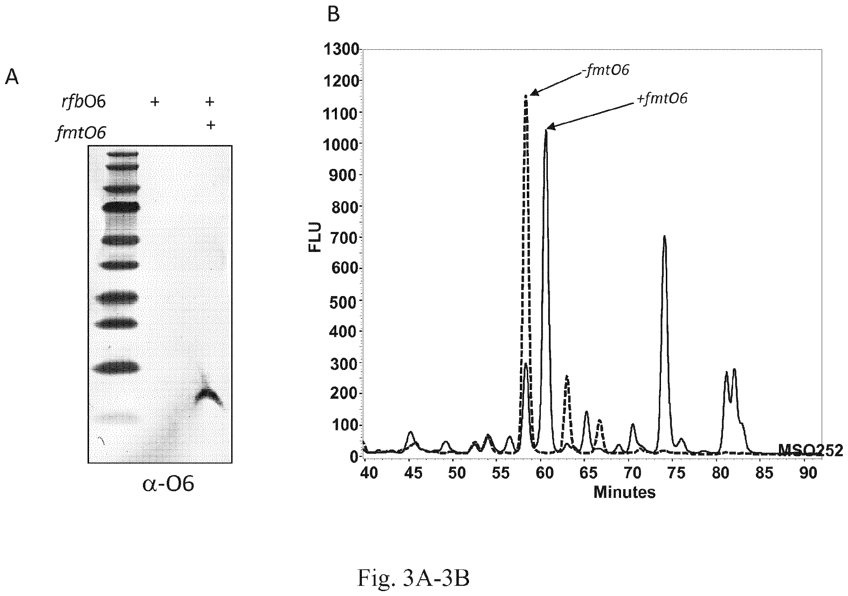

Figure 3. Functional testing of *Pseudomonas aeruginosa* O6 formyltransferase. 3A: Detection of formylated single O6 repeat unit bound to lipid A core by Western blotting. *E*. *coli* W3110 Δwec was transformed with a cosmid encoding the (incomplete) rfbO6 cluster and an expression plasmid encoding the O6 formyltransferase (*fmtO6;* SEQ NO:2). Cell extracts were harvested after overnight induction during growth at 37°C in LB medium, digested with proteinase K, separated by SDS PAGE, and electroblotted on nitrocellulose membranes. Immunodetection with an O6 specific antiserum induced a signal in the presence of fmtO6, but not in the empty vector control. This result strongly indicates that formylation is a relelvant antigen on *P. aeruginosa* cells and a prerequisite for detection using this antiserum. **3B:** Confirmation of formylation on a single O6 repeat unit released from undecaprenylpyrophosphate. *E*. *coli* W3110 Δwec ΔwaaL was transformed with the same plamids as above and grown in shake flasks to produce O6 O antigen single repeat units (the wzy polymerase is missing in these strains) and glycolipids were analyzed. Briefly, repeat units were extracted as glycolipids from dried cells, purified by affinity to C18 cartridges, hydrolyzed (to remove undecaprenylpyrophosphate from the O6 O antigen repeat units), labelled with 2 aminobenzamide using reductive amination, and analyzed by normal phase HPLC. Coexpression of fmt06 gave rise to an additional signal at 61' elution time, containing oligosaccharides corresponding to the labelled, formylated O6 repeat unit, whereas in absence of the gene, the main signal was found at 58' and contained the labelled N acetylated O6 repeat unit.

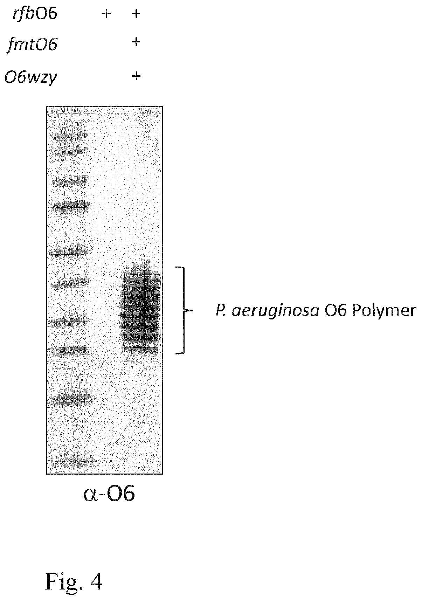

Figure 4. Functional testing of *P. aeruginosa* O6 candidate wzy polymerase. *E*. *coli* W3110 Δwec cells containing a cosmid encoding the (incomplete) rfb cluster (lacking the *fmtO6* and *wzy* genes) was transformed with plasmids encoding the *fmtO6* and *wzy* candidate gene PAK_01823 (SEQ ID NO:3) or corresponding empty vectors. Cell extracts were treated with proteinase K and LPS analyzed by immunodetection after SDS PAGE and electrotransfer to nitrocellulose membranes.

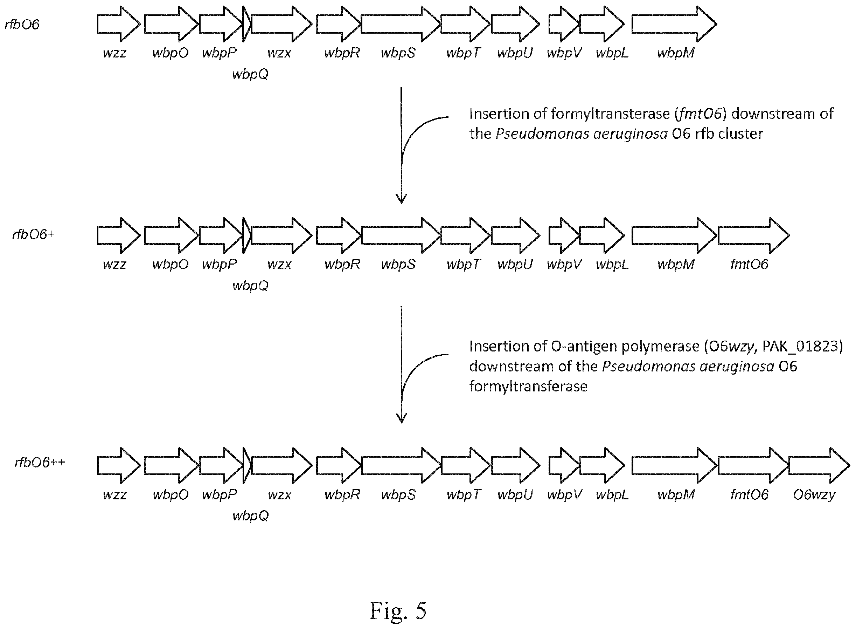

Figure 5. Cloning of the artificial *Pseudomonas aeruginosa* O6 O antigen expression cluster. First, the rfb cluster of *P. aeruginosa* O6 strain stGVXN4017 (*Pseudomonas aeruginosa* O6 "PAK" strain) was cloned into a cosmid vector by PCR cloning using standard techniques. Bioinformatics supported homology searches identified the formyltransferase (FT) and O-antigen polymerase (*wzy*), which were subsequently inserted downstream of the rfb cluster in a step wise manner. The resulting gene clusters are able to commit complete *P. aeruginosa* O6 O antigen repeat unit biosynthesis (rfbO6+, no polymer) and polysaccharide (rfbO6++, in which wzy is included) biosynthesis in *E*. *coli* W3110 derivatives.

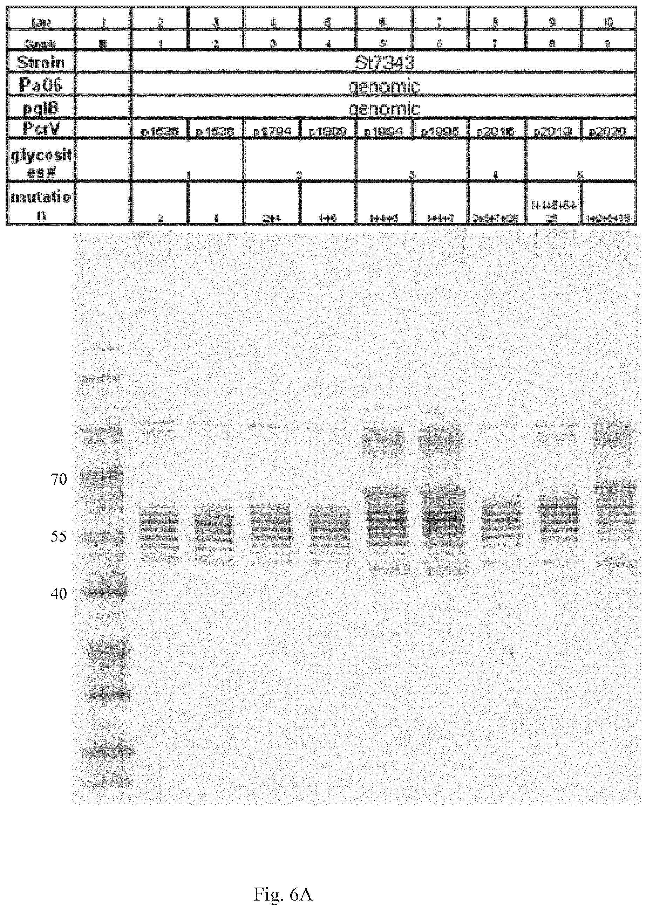

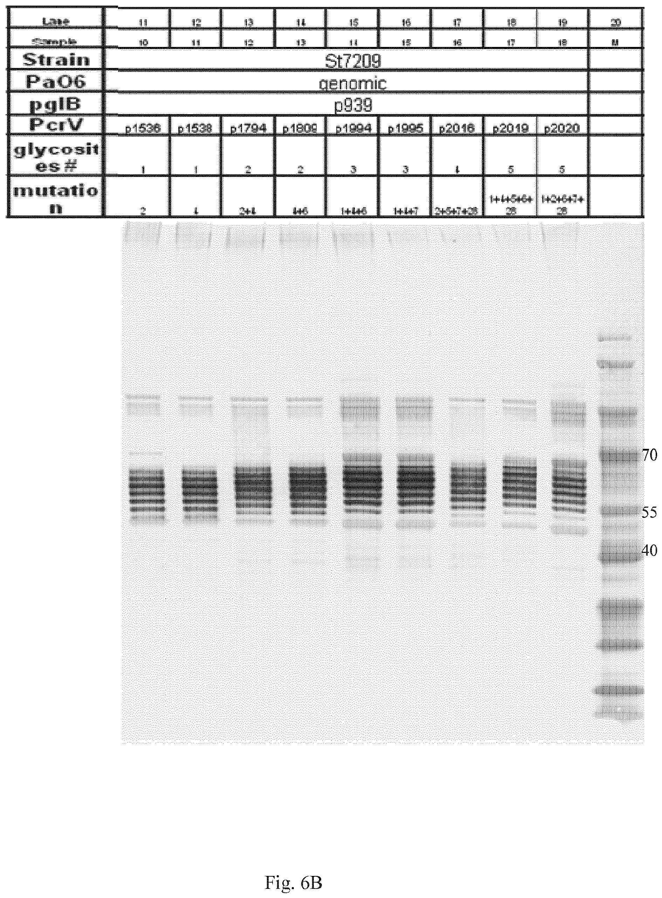

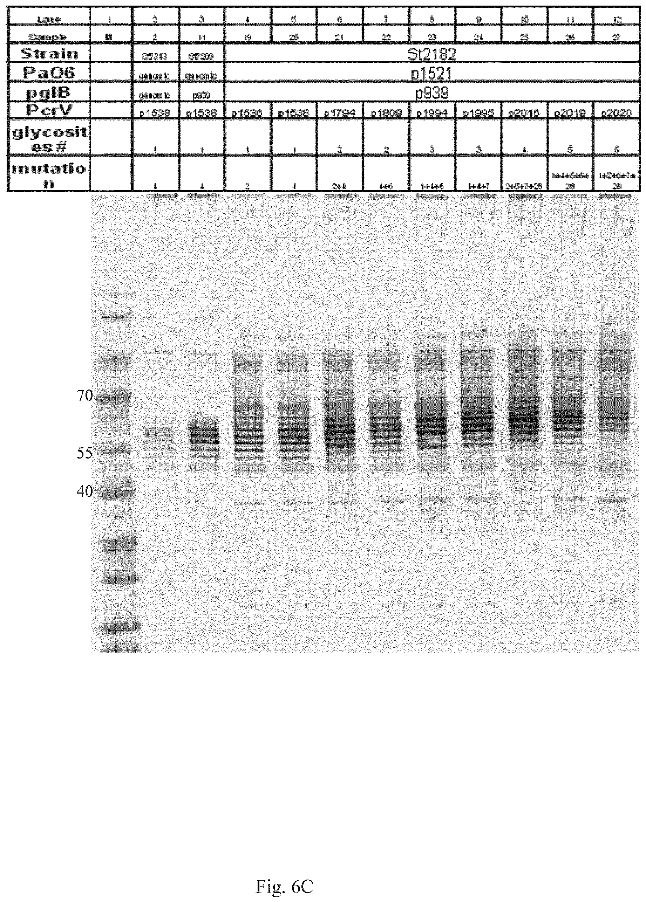

Fig. 6 depicts a Western blot of periplasmic extracts from modified host cells that produce bioconjugates. Strains as described in the Examples are indicated. **6A:** results for "St7343" *E*. *coli* strain modified to comprise integrated *pglB* and integrated *rfb* cluster from *P. aeruginosa* O6. **6B:** results for "St7209" *E*. *coli* strain modified to comprise plasmid-borne *pglB* and integrated *rfb* cluster from *P. aeruginosa* O6. **6C:** results for "St2182" *E*. *coli* strain modified to comprise plasmid-borne *pglB* and plasmid-borne *rfb* cluster from *P. aeruginosa* O6.

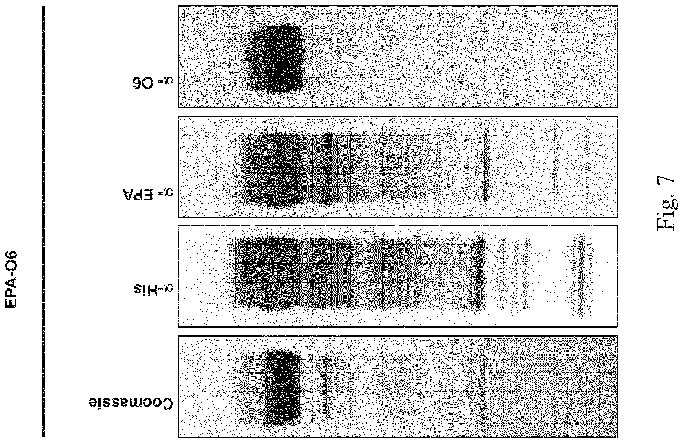

Figure 7. Purified EPA-06 glycoconjugate. EPA-06 was purified from periplasmic extract of modified host cells using Metal-chelate affinity chromatography, anion exchange chromatography and size exclusion chromatography (SEC). The final SEC eluate was characterized by SDS-PAGE followed by Coomassie Blue staining or Western blot using the indicated antibodies.

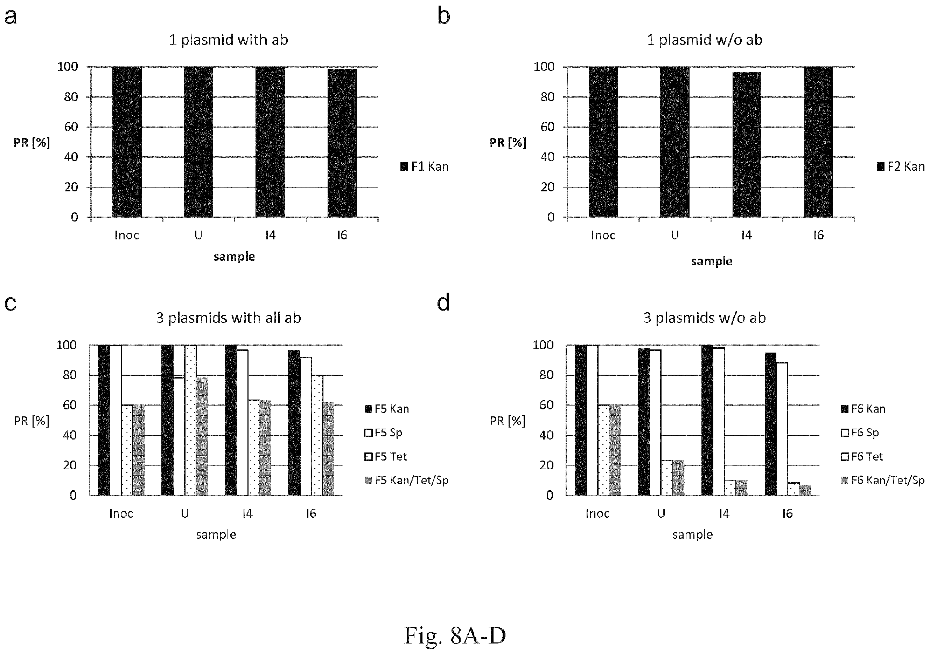

Figure 8. Plasmid retention (PR) of 1 and 3 plasmid systems in the presence and absence of antibiotic selection pressure. The PR is expressed in % of cells that contain the respective plasmid. Figures A and B show PR of the EPA-plasmid (Kanamycin, black) in modified host cells with integrated rfb cluster and *pglB* in the presence (A) and absence (B) of Kanamycin. Figures C and D show PR of the EPA-plasmid (Kanamycin, black), *pglB-*Plasmid (Spectinomycin, white) and rfb cluster plasmid (Tetracyclin, dotted) in modified host cells in the presence (C) and absence (D) of all three antibiotics. The percentage of cells in which all three plasmids are retained is shown in grey. Inoc=Inoculum; U=uninduced cells; I4=cells 6hours after induction; I6=cells after o/n induction

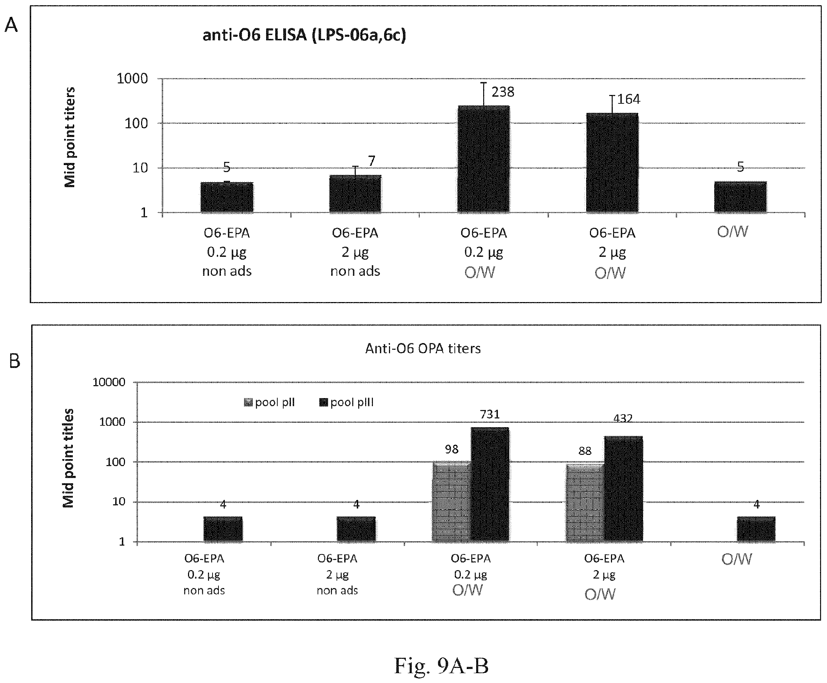

Figure 9. Biologic activity of vaccine induced anti-O6 antiserum. **9A:** ELISA mid point titers of pooled mouse sera from the different vaccination groups after the third injection. Non ads= non adjuvanted, O/W: indicates the adjuvant used, an oil-in-water emulsion adjuvant. O/W alone is a control group that did not contain a glycoconjugate. **9B:** Opsonophagocytotic killing mid point titers (inducing a 50% reduction in cfu compared to control) are indicated. Pool pII and pIII are pooled sera harvested after the second and third injection.

### 5. DETAILED DESCRIPTION

### 5.1 Host Cells

Provided herein are host cells, e.g., prokaryotic host cells, capable of producing bioconjugates comprising a *Pseudomonas* antigen linked to a carrier protein. The host cells described herein comprise a genome into which one or more DNA sequences has been inserted, wherein said DNA sequences encode a protein or comprise an operon involved in glycosylation of proteins, e.g., N-glycosylation of proteins. For example, in certain embodiments, a host cell described herein comprises a genome into which one or more of the following has been inserted: DNA encoding an oligosaccharyl transferase, DNA encoding a glycosyltransferase, DNA encoding a carrier protein, DNA comprising an *rfb* gene cluster, DNA comprising a capsular polysaccharide gene cluster, DNA encoding a flippase, DNA encoding an epimerase, DNA encoding a protein associated with a capsular polysaccharide gene cluster, DNA encoding a protein involved in capsular polysaccharide assembly, DNA encoding a protein involved in O antigen assembly, DNA encoding a protein involved in lipopolysaccharide assembly, and/or DNA encoding a protein involved in lipooligosaccharide assembly. In a specific embodiment, the host cell is *E*. *coli.*

### Host Cells that Produce Pseudomonas Bioconjugates

In another aspect, provided herein is a modified prokaryotic host cell comprising nucleic acids encoding enzymes capable of producing a bioconjugate comprising a *Pseudomonas* antigen. Such host cells may naturally express nucleic acids specific for production of a *Pseudomonas* antigen, or the host cells may be made to express such nucleic acids, *i.e.,* in certain embodiments said nucleic acids are heterologous to the host cells. In certain embodiments, one or more of said nucleic acids specific for production of a *Pseudomonas* antigen are heterologous to the host cell and intergrated into the genome of the host cell. In certain embodiments, the host cells provided herein comprise nucleic acids encoding additional enzymes active in the N-glycosylation of proteins, e.g., the host cells provided herein further comprise a nucleic acid encoding an oligosaccharyl transferase and/or one or more nucleic acids encoding other glycosyltransferases. In certain embodiments, the host cells provided herein comprise a nucleic acid encoding a carrier protein, *e.g.,* a protein to which oligosaccharides and/or polysaccharides can be attached to form a bioconjugate. In a specific embodiment, the host cell is *E*. *coli.*

In a specific embodiment, provided herein is a modified prokaryotic host cell comprising nucleic acids encoding enzymes capable of producing a bioconjugate comprising a *Pseudomonas* antigen, wherein said host cell comprises an *rfb* cluster from *Pseudomonas* or a glycosyltransferase derived from an *rfb* cluster from *Pseudomonas.* In a specific embodiment, said *rfb* cluster from *Pseudomonas* or glycosyltransferase derived from an *rfb* cluster from *Pseudomonas* is integrated into the genome of said host cell. In another specific embodiment, said *rfb* cluster from *Pseudomonas* or glycosyltransferase derived from an *rfb* cluster from *Pseudomonas* is an *rfb* cluster from *Pseudomonas aeruginosa.* In another specific embodiment, said host cell comprises a nucleic acid encoding an oligosaccharyl transferase (*e.g., pglB* from *Campylobacter jejuni*). In another specific embodiment, said nucleic acid encoding an oligosaccharyl transferase (*e.g., pglb* from *Campylobacter jejuni*) is integrated into the genome of the host cell. In a specific embodiment, said host cell comprises a nucleic acid encoding a carrier protein. In another specific embodiment, the host cell is *E*. *coli.*

In another specific embodiment, provided herein is a modified prokaryotic host cell comprising (i) an *rfb* cluster from *Pseudomonas,* wherein said *rfb* cluster is integrated into the genome of said host cell; (ii) a nucleic acid encoding an oligosaccharyl transferase (*e.g.,pglB* from *Campylobacter jejuni*), wherein said nucleic acid encoding an oligosaccharyl transferase is integrated into the genome of the host cell; and (iii) a carrier protein, wherein said carrier protein is either plasmid-borne or integrated into the genome of the host cell. In another specific embodiment, said *rfb* cluster from *Pseudomonas* is an *rfb* cluster from *Pseudomonas aeruginosa.* In another specific embodiment, the host cell is *E*. *coli.*

In another specific embodiment, provided herein is a modified prokaryotic host cell comprising (i) a glycosyltransferase derived from an *rfb* cluster from *Pseudomonas,* wherein said glycosyltransferase is integrated into the genome of said host cell; (ii) a nucleic acid encoding an oligosaccharyl transferase (*e.g.,pglB* from *Campylobacter jejuni*), wherein said nucleic acid encoding an oligosaccharyl transferase is integrated into the genome of the host cell; and (iii) a carrier protein, wherein said carrier protein is either plasmid-borne or integrated into the genome of the host cell. In another specific embodiment, said glycosyltransferase derived from an *rfb* cluster from *Pseudomonas* is an *rfb* cluster from *Pseudomonas aeruginosa.* In another specific embodiment, the host cell is *E*. *coli.*

In a specific embodiment, the *rfb* cluster from *Pseudomonas* or glycosyltransferase derived from an *rfb* cluster from *Pseudomonas* is an *rfb* cluster or glycosyltransferase from *Pseudomonas aeruginosa.* In another specific embodiment, said *rfb* cluster from *Pseudomonas* or glycosyltransferase derived from an *rfb* cluster from *Pseudomonas* is an *rfb* cluster or glycosyltransferase from *Pseudomonas aeruginosa* serotype O1, O2, O3, O4, O5, O6, O7, O8, O9, O10, O11, 012, 013, 014, 015, 016, 017, 018, 019, or O20. In another specific embodiment, said *rfb* cluster from *Pseudomonas aeruginosa* is the *rfb* cluster from any one of the serotypes described in Knirel et al., 2006, Journal of Endotoxin Research 12(6): 324-336. In a specific embodiment, said *rfb* cluster from *Pseudomonas* or glycosyltransferase derived from an *rfb* cluster from *Pseudomonas* is an *rfb* cluster or glycosyltransferase from *Pseudomonas aeruginosa* serotype O6 PAK strain. In a specific embodiment, said *rfb* cluster from *Pseudomonas* or glycosyltransferase derived from an *rfb* cluster from *Pseudomonas* is an *rfb* cluster or glycosyltransferase from *Pseudomonas aeruginosa* serotype O11, *e.g., Pseudomonas aeruginosa* strain PA103 (see, e.g., Genbank Accession No. KF364633.1). In a specific embodiment, the genes encoding a formyltransferase enzyme (GenBank: EOT23134.1; NCBI protein ID: PAK_01412; SEQ ID NO:2) and a wzy polymerase (GenBank: EOT19368.1; NCBI protein ID: PAK 01823; SEQ ID NO:3) are introduced (e.g., via plasmid or integration) in addition to said *rfb* cluster from *Pseudomonas aeruginosa* serotype O6 PAK strain in order to functionally extend it.

In a specific embodiment, a modified prokaryotic host cell provided herein comprises a nucleic acid that encodes a formyltransferase. In another specific embodiment, said formyltransferase is the formyltransferase presented in SEQ ID NO:2, or a homolog thereof. In another specific embodiment, said formyltransferase is incorporated (e.g., inserted into the genome of or plasmid expressed by) in said host cell as part of a *Pseudomonas rfb* cluster, wherein said *Pseudomonas rfb* cluster has been modified to comprise the formyltransferase. In another specific embodiment, said *Pseudomonas rfb* cluster is a *Pseudomonas aeruginosa* serotype O6 *rfb* cluster.

In another specific embodiment, a modified prokaryotic host cell provided herein comprises a nucleic acid that encodes a wzy polymerase. In another specific embodiment, said wzy polymerase is the wzy polymerase presented in SEQ ID NO:3, or a homolog thereof. In another specific embodiment, said wzy polymerase is incorporated (e.g., inserted into the genome of or plasmid expressed by) in said host cell as part of a *Pseudomonas rfb* cluster, wherein said *Pseudomonas rfb* cluster has been modified to comprise the wzy polymerase. In another specific embodiment, said *Pseudomonas rfb* cluster is a *Pseudomonas aeruginosa* serotype O6 *rfb* cluster.

In another specific embodiment, a modified prokaryotic host cell provided herein comprises (i) a nucleic acid that encodes a formyltransferase and (ii) a nucleic acid that encodes a wzy polymerase. In a specific embodiment, said formyltransferase is the formyltransferase presented in SEQ ID NO:2, or a homolog thereof. In another specific embodiment, said wzy polymerase is the wzy polymerase presented in SEQ ID NO:3, or a homolog thereof. In a specific embodiment, said formyltransferase and said wzy polymerase are incorporated (e.g., inserted into the genome of or plasmid expressed by) in said host cell as part of a *Pseudomonas rfb* cluster, wherein said *Pseudomonas rfb* cluster has been modified to comprise the formyltransferase and wzy polymerase. In another specific embodiment, said *Pseudomonas rfb* cluster is a *Pseudomonas aeruginosa* serotype O6 *rfb* cluster.

Nucleic acids that encode formyltransferases and nucleic acids that encode wzy polymerases that are use to generate modified *Pseudomonas rfb* clusters, e.g., modified *Pseudomonas aeruginosa* serotype O6 *rfb* clusters, can be inserted into the *rfb* cluster at multiple positions and in multiple orientations.

In a specific embodiment, the gene encoding said formyltransferase and/or the gene encoding said wzy polymerase is/are inserted downstream of the genes of the *Pseudomonas rfb* cluster, e.g., the *Pseudomonas aeruginosa* serotype O6 *rfb* cluster. In a specific emnodiment, the gene encoding said formyltransferase and/or the gene encoding said wzy polymerase is/are inserted downstream of the *wbpM* gene of the *Pseudomonas aeruginosa* serotype O6 *rfb* cluster.

In a specific embodiment, the gene encoding said formyltransferase and/or the gene encoding said *wzy* polymerase is/are inserted upstream of the genes of the *Pseudomonas rfb* cluster, e.g., the *Pseudomonas aeruginosa* serotype O6 *rfb* cluster. In a specific emnodiment, the gene encoding said formyltransferase and/or the gene encoding said *wzy* polymerase is/are inserted downstream of the wzz gene of the *Pseudomonas aeruginosa* serotype O6 *rfb* cluster.

In a specific embodiment, the gene encoding said formyltransferase and/or the gene encoding said wzy polymerase is/are inserted in a clockwise orientation relative to the genes of the *Pseudomonas rfb* cluster, e.g., the *Pseudomonas aeruginosa* serotype O6 *rfb* cluster.

In a specific embodiment, the gene encoding said formyltransferase and/or the gene encoding said *wzy* polymerase is/are inserted in a counter-clockwise orientation relative to the genes of the *Pseudomonas rfb* cluster, e.g., the *Pseudomonas aeruginosa* serotype O6 *rfb* cluster.

In a specific embodiment, provided herein is a modified prokaryotic host cell comprising nucleic acids encoding enzymes capable of producing a bioconjugate comprising a *Pseudomonas* O6 antigen. In a specific embodiment, said host cell comprises the *Pseudomonas aeruginosa* serotype O6 rfb cluster, a nucleic acid encoding a wzy polymerase, and a formyltransferase. In a specific embodiment, the wzy polymerase is the *P. aeruginosa* O6 wzy polymerase (SEQ ID NO:3), or a homolog thereof (e.g., the *wzy* polymerase from the PAK or LESB58 strain of *Pseudomonas aeruginosa*). In another specific embodiment, the formyltransferase is the *P. aeruginosa* O6 formyltransferase (SEQ ID NO:2), or a homolog thereof (e.g., the formyltransferase from the PAK or LESB58 strain of *Pseudomonas aeruginosa*). In certain embodiments, one or more of the nucleic acids encoding the rfb cluster, the wzy polymerase, and/or the formyltransferase are inserted into the genome of the host cell, e.g., using a method described herein. In a specific embodiment, each of the nucleic acids encoding the rfb cluster, the wzy polymerase, and the formyltransferase are inserted into the genome of the host cell, e.g., using a method described herein. In certain embodiments, the host cell further comprises a nucleic acid encoding an oligosaccharyl transferase (*e.g., pglB* from *Campylobacter jejuni*), wherein said nucleic acid encoding an oligosaccharyl transferase is either plasmid-borne or integrated into the genome of the host cell; and a nucleic acid encoding a carrier protein, wherein said nucleic acid encoding said carrier protein is either plasmid-borne or integrated into the genome of the host cell. In a specific embodiment, said nucleic acid encoding said oligosaccharyl transferase is integrated into the genome of the host cell.

### Genetic Background

Exemplary host cells that can be used to generate the modified host cells described herein include, without limitation, *Escherichia* species, *Shigella* species, *Klebsiella* species, *Xhantomonas* species, *Salmonella* species, *Yersinia* species, *Lactococcus* species, *Lactobacillus* species, *Pseudomonas* species, *Corynebacterium* species, *Streptomyces* species, *Streptococcus* species, *Staphylococcus* species, *Bacillus* species, and *Clostridium* species. In a specific embodiment, the host cell used herein is *E*. *coli.*

In certain embodiments, the host cell genetic background is modified by, e.g., deletion of one or more genes. Exemplary genes that can be deleted in host cells (and, in some cases, replaced with other desired nucleic acid sequences) include genes of host cells involved in glycolipid biosynthesis, such as *waaL* (see, e.g., Feldman et al., 2005, PNAS USA 102:3016-3021), the O antigen cluster (*rfb* or *wb*), enterobacterial common antigen cluster (*wec*), the lipid A core biosynthesis cluster (*waa*), and prophage O antigen modification clusters like the *gtrABS* cluster. In a specific embodiment, the host cells described herein are modified such that they do not produce any O antigens other than a desired O antigen from, *e.g.,* an O antigen *Pseudomonas.* In a specific embodiment, one or more of the *waaL* gene, *gtrA* gene, *gtrB* gene, *gtrS* gene, or a gene or genes from the wec cluster or a gene or genes from the *rfb* gene cluster are deleted or functionally inactivated from the genome of a prokaryotic host cell provided herein. In one embodiment, a host cell used herein is *E*. *coli,* wherein the *waaL* gene, *gtrA* gene, *gtrB* gene, *gtrS* gene are deleted or functionally inactivated from the genome of the host cell. In another embodiment, a host cell used herein is *E*. *coli,* wherein the *waaL* gene and *gtrS* gene are deleted or functionally inactivated from the genome of the host cell. In another embodiment, a host cell used herein is *E*. *coli,* wherein the *waaL* gene and genes from the wec cluster are deleted or functionally inactivated from the genome of the host cell.

### Carrier Proteins

Any carrier protein suitable for use in the production of conjugate vaccines (*e.g*., bioconjugates for use in vaccines) can be used herein, e.g., nucleic acids encoding the carrier protein can be introduced into a host provided herein for the production of a bioconjugate comprising a carrier protein linked to *Pseudomonas* antigen. Exemplary carrier proteins include, without limitation, detoxified Exotoxin A of *P. aeruginosa* (EPA; see, e.g., Ihssen, et al., (2010) Microbial cell factories 9, 61), CRM197, maltose binding protein (MBP), Diphtheria toxoid, Tetanus toxoid, detoxified hemolysin A of *S. aureus,* clumping factor A, clumping factor B, *E*. *coli* FimH, *E*. *coli* FimHC, *E*. *coli* heat labile enterotoxin, detoxified variants of *E. coli* heat labile enterotoxin, Cholera toxin B subunit (CTB), cholera toxin, detoxified variants of cholera toxin, *E*. *coli* Sat protein, the passenger domain of *E. coli* Sat protein, *Streptococcus pneumoniae* Pneumolysin and detoxified variants thereof, *C. jejuni* AcrA, *Pseudomonas* PcrV protein, and *C. jejuni* natural glycoproteins.

In specific embodiments, the carrier proteins expressed by the modified host cells provided herein are expressed from a nucleic acid that has been integrated into the genome of the modified host cell. That is, a nucleic acid encoding the carrier protein has been integrated into the host cell genome. In certain embodiments, the carrier proteins expressed by the modified host cells provided herein are expressed from a plasmid that has been introduced into the modified host cell.

In certain embodiments, the carrier proteins used in the generation of the bioconjugates described herein are modified, e.g., modified in such a way that the protein is less toxic and/or more susceptible to glycosylation. In a specific embodiment, the carrier proteins used in the generation of the bioconjugates described herein are modified such that the number of glycosylation sites in the carrier proteins is maximized in a manner that allows for lower concentrations of the protein to be administered, e.g., in an immunogenic composition, in its bioconjugate form.

In certain embodiments, the carrier proteins described herein are modified to include 1, 2, 3, 4, 5, 6, 7, 8, 9, 10, or more glycosylation sites than would normally be associated with the carrier protein (e.g., relative to the number of glycosylation sites associated with the carrier protein in its native/natural, e.g., "wild-type" state). In specific embodiments, introduction of glycosylation sites is accomplished by insertion of glycosylation consensus sequences (e.g., Asn-X-Ser(Thr), wherein X can be any amino acid except Pro; or Asp(Glu)-X-Asn-Z-Ser(Thr), wherein X and Z are independently selected from any natural amino acid except Pro (see WO 2006/119987)) anywhere in the primary structure of the protein. Introduction of such glycosylation sites can be accomplished by, e.g., adding new amino acids to the primary structure of the protein (i.e., the glycosylation sites are added, in full or in part), or by mutating existing amino acids in the protein in order to generate the glycosylation sites (i.e., amino acids are not added to the protein, but selected amino acids of the protein are mutated so as to form glycosylation sites). Those of skill in the art will recognize that the amino acid sequence of a protein can be readily modified using approaches known in the art, e.g., recombinant approaches that include modification of the nucleic acid sequence encoding the protein. In specific embodiments, glycosylation consensus sequences are introduced into specific regions of the carrier protein, e.g., surface structures of the protein, at the N or C termini of the protein, and/or in loops that are stabilized by disulfide bridges at the base of the protein. In certain embodiments, the classical 5 amino acid glycosylation consensus sequence may be extended by lysine residues for more efficient glycosylation, and thus the inserted consensus sequence may encode 5, 6, or 7 amino acids that should be inserted or that replace acceptor protein amino acids.

In certain embodiments, the carrier proteins used in the generation of the bioconjugates described herein comprise a "tag," i.e., a sequence of amino acids that allows for the isolation and/or identification of the carrier protein. For example, adding a tag to a carrier protein described herein can be useful in the purification of that protein and, hence, the purification of conjugate vaccines comprising the tagged carrier protein. Exemplary tags that can be used herein include, without limitation, histidine (HIS) tags (e.g., hexa histidine-tag, or 6XHis-Tag), FLAG-TAG, and HA tags. In certain embodiments, the tags used herein are removable, e.g., removal by chemical agents or by enzymatic means, once they are no longer needed, e.g., after the protein has been purified.

In certain embodiments, the carrier proteins described herein comprise a signal sequence that targets the carrier protein to the periplasmic space of the host cell that expresses the carrier protein. In a specific embodiment, the signal sequence is from *E*. *coli* DsbA, *E*. *coli* outer membrane porin A (OmpA), *E*. *coli* maltose binding protein (MalE), *Erwinia carotovorans* pectate lyase (PelB), FlgI, NikA, or *Bacillus* sp. endoxylanase (XynA), heat labile *E*. *coli* enterotoxin LTIIb, *Bacillus* endoxylanase XynA, or *E*. *coli* flagellin (FlgI).

### Glycosylation Machinery

### Oligosaccharyl Transferases

Oligosaccharyl transferases transfer lipid-linked oligosaccharides to asparagine residues of nascent polypeptide chains that comprise an N-glycoxylation consensus motif, e.g., Asn-X-Ser(Thr), wherein X can be any amino acid except Pro; or Asp(Glu)-X-Asn-Z-Ser(Thr), wherein X and Z are independently selected from any natural amino acid except Pro (see WO 2006/119987). See, e.g., WO 2003/074687 and WO 2006/119987.

In certain embodiments, the host cells provided herein comprise a nucleic acid that encodes an oligosaccharyl transferase. The nucleic acid that encodes an oligosaccharyl transferase can be native to the host cell, or can be introduced into the host cell using genetic approaches, as described above. The oligosaccharyl transferase can be from any source known in the art. In a specific embodiment, the oligosaccharyl transferase is an oligosaccharyl transferase from *Campylobacter.* In another specific embodiment, the oligosaccharyl transferase is an oligosaccharyl transferase from *Campylobacter jejuni* (i.e., *pglB;* see, e.g., Wacker et al., 2002, Science 298:1790-1793; see also, e.g., NCBI Gene ID: 3231775, UniProt Accession No. 086154). In another specific embodiment, the oligosaccharyl transferase is an oligosaccharyl transferase from *Campylobacter lari* (see, e.g., NCBI Gene ID: 7410986).

In a specific embodiment, the modified host cells provided herein comprise a nucleic acid sequence encoding an oligosaccharyl transferase, wherein said nucleic acid sequence encoding an oligosaccharyl transferase is integrated into the genome of the host cell.

### Accessory Enzymes

In certain embodiments, nucleic acids encoding one or more accessory enzymes are introduced into the modified host cells described herein. Such nucleic acids encoding one or more accessory enzymes can be either plasmid-borne or integrated into the genome of the host cells described herein. Exemplary accessory enzymes include, without limitation, epimerases, branching, modifying, amidating, chain length regulating, acetylating, formylating, polymerizing enzymes.

Nucleic acid sequences encoding epimerases that can be inserted into the host cells described herein are known in the art. In certain embodiments, the epimerase inserted into a host cell described herein is an epimerase described in International Patent Application Publication No. WO 2011/062615. In a specific embodiment, the epimerase is the epimerase encoded by the Z3206 gene of *E. coli* strain 0157. See, e.g., WO 2011/062615 and Rush et al., 2009, The Journal of Biological Chemistry 285:1671-1680. In a specific embodiment, the modified host cells provided herein comprise a nucleic acid sequence encoding an epimerase, wherein said nucleic acid sequence encoding an epimerase is integrated into the genome of the host cell.

In certain embodiments, a nucleic acid sequence encoding a formyltransferase is inserted into or expressed by the host cells described herein. Formyltransferases are enzymes that catalyse the transfer of a formyl group to an acceptor molecule. In a specific embodiment, a nucleic acid sequence encoding the *Pseudomonas aeruginosa* O6 formyltransferase fmtO6 (SEQ ID NO:2), or a homolog thereof (e.g., the *wzy* polymerase from the PAK or LESB58 strain of *Pseudomonas aeruginosa*), is inserted into or expressed by the host cells described herein. In another specific embodiment, a nucleic acid sequence that encodes a protein having about or at least 80%, 85%, 90%, 95%, 96%, 97%, 98%, or 99% identity or homology to SEQ ID NO:2 is inserted into or expressed by the host cells described herein.

Certain formyltransferases involved in polysaccharide biosynthesis are known, and can be inserted into or expressed by the host cells described herein . For example, *vioF* is an enzyme from *P. alcalifaciens* serotype O30, which is 48% identical to the formyltransferase from *Francisella tularensis* (Nagaraja et al. 2005). It converts dTDP-D-Qui4N to dTDP-D-Qui4NFo, and is involved in O-antigen biosynthesis (Liu et al. 2012, Glycobiology 22(9):1236-1244). Another formyltransferase involved in polysaccharide biosynthesis is *arnA* (e.g., from *E. coli*), a bifunctional enzyme in which the N-terminal domain converts UDP-Ara4N to UDP-AraNFo, while the C-terminal domain is involved in oxidative decarboxylation of UDP-glucuronic acid. Both enzymatic activities are required for L-Ara4N modification of LipidA and polymyxin resistance (Breazeale et al., 2005, The Journal of Biological Chemistry 280(14):14154-14167). Another formyltransferase involved in polysaccharide biosynthesis is *wekD,* an enzyme from *E*. *coli* serotype 0119, involved in the biosynthesis of TDP-DRhaNAc3NFo (Anderson et al., 1992, Carbohydr Res. 237:249-62).

Further, domains that are related to formyltransferase activity have been characterized. The so called FMT_core domain is present in the majority of formyltransferases. Examples include the methionyl-tRNA formyltransferase, phosphoribosylglycinamide formyltransferase 1, UDP-glucuronic acid decarboxylase/UDP-4-amino-4-deoxy-L-arabinose formyltransferase, *vioF* from *Providencia alcalifaciens* O30, and *arnA* from *E.coli.* The above mentioned formyltransferases use FTHF (N-10-formyltetrahydrofolate) as formyl donor. Also, formate producing enzymes using FTHF (10-formyltetrahydrofolate) as substrate contain this domain. In addition, AICARFT is present in phosphoribosylaminoimidazolecarboxamide formyltransferase/IMP cyclohydrolase and FDH_GDH is present in phosphoribosylglycinamide formyltransferase 2.

In certain embodiments, a nucleic acid sequence encoding an O antigen polymerase (wzy gene) is inserted into or expressed by the host cells described herein. O antigen polymerases are multi spanning transmembrane proteins. They use undecaprenylpyrophosphate bound O antigen repeat units as substrates to generate a linear polymer constisting of the repeat units. O antigen polymerases (*wzy*) are present in Gram negative bacteria that synthesize O antigen polymers via a *wzy* dependent mechanism.

In a specific embodiment, a nucleic acid sequence encoding the *Pseudomonas aeruginosa wzy* polymerase (SEQ ID NO:3), or a homolog thereof (e.g., the *wzy* polymerase from the PAK or LESB58 strain of *Pseudomonas aeruginosa*), is inserted into or expressed by the host cells described herein. Examples of bacteria known to comprise wzy polymerases include *Escherichia coli, Pseudomonas aeruginosa, Shigella flexneri* and *Salmonella typhimurium.* In another specific embodiment, a nucleic acid sequence that encodes a protein having about or at least 80%, 85%, 90%, 95%, 96%, 97%, 98%, or 99% identity or homology to SEQ ID NO:3 is inserted into or expressed by the host cells described herein.

### Gene Copy Number

In certain embodiments, the copy number of a gene(s) integrated into a modified host cell provided herein is 1, 2, 3, 4, 5, 6, 7, 8, 9, or 10. In a specific embodiment, the copy number of a gene(s) integrated into a modified host cell provided herein is 1 or 2.

### Benefits

The modified host cells described herein are of particular commercial importance and relevance, as they allow for large scale fermentation of bioconjugates comprising *Pseudomonas* antigens that can be used as therapeutics (e.g., in immunogenic compositions, vaccines), at a lower risk due to the increased stability of the chromosomally inserted DNA and thus expression of the DNA of interest during fermentation. The modified host cells described herein are advantageous over host cells that rely on plasmid borne expression of nucleic acids required for generation of the bioconjugates described herein because, *inter alia,* antibiotic selection during fermentation is not required once the heterologous DNA is inserted into the host cell genome. That is, when the insert DNA is inserted in the chromosome, it doesn't need to be selected for, because it is propagated along with replication of the host genome. Further, it is a known disadvantage in plasmid borne systems that with every generation (i.e., cycle of host cell replication) the risk for losing the plasmid increases. This loss of plasmid is due to the sometimes inappropriate distribution of plasmids to daughter cells at the stage of cell separation during cell division. At large scale, bacterial cell cultures duplicate more often than in smaller fermentation scales to reach high cell densities. Thus, higher cell stability and insert DNA expression leads to higher product yields, providing a distinct advantage. Cell stability is furthermore a process acceptance criteria for approval by regulatory authorities, while antibiotic selection is generally not desired during fermentation for various reasons, e.g., antibiotics present as impurities in the final medicial products and bear the risk of causing allergic reactions, and antibiotics may promote antibiotic resistance (e.g., by gene transfer or selection of resistant pathogens).

The present application provides modified host cells for use in making bioconjugates comprising *Pseudomonas* antigens that can be used as therapeutics (e.g., in immunogenic compositions, vaccines), wherein certain genetic elements required to drive the production of bioconjugates are integrated stably into the host cell genome. Consequently the host cell can contain a reduced number of plasmids, just a single plasmid or no plasmids at all. In some embodiments, the presence of a single plasmid can result in greater flexibility of the production strain and the ability to change the nature of the conjugation (in terms of its saccharide or carrier protein content) easily leading to greater flexibility of the production strain.

In general, a reduction in the use of plasmids leads to a production strain which is more suited for use in the production of medicinal products. A drawback of essential genetic material being present on plasmids is the requirement for selection pressure to maintain the episomal elements in the host cell. The selection pressure requires the use of antibiotics, which is undesirable for the production of medicinal products due to, e.g., the danger of allergic reactions against the antibiotics and the additional costs of manufacturing. Furthermore, selection pressure is often not complete, resulting in inhomogeneous bacterial cultures in which some clones have lost the plasmid and thus are not producing the bioconjugate. The host cells described herein therefore are able to produce a safer product that can be obtained in high yields.

### 5.2 Methods of Integration/Introduction of Nucleic Acids into Host Cells

Any method known in the art can be used to integrate a nucleic acid (e.g., a gene or an operon, e.g., *rfb* cluster) into the genome of a host cell.

In a specific embodiment, heterologous nucleic acids are introduced into the host cells described herein using the method of insertion described in International Patent application No. PCT/EP2013/071328. According to this method, large contiguous sequences of DNA can be stably inserted directly into a host cell genome. Briefly, the method comprises some or all of following steps:

Step 1: A donor plasmid is made. A desired heterologous insert DNA sequence (i.e., a heterologous insert DNA sequence that comprises one or more genes of interest) is cloned into a cloning site (e.g., a multiple cloning site, abbreviated as MCS) of a plasmid suitable for use as a donor plasmid. DNA sequences suitable for use as homology regions (i.e., DNA sequences homologous to the insertion location on the host cell genome) also are cloned into the donor plasmid, such that the homology regions flank the heterologous insert DNA. These methods of cloning and assembly of the donor plasmid can be done according to any established and well known technology to modify and synthesize DNA such as, without limitation, molecular cloning using restriction enzymes and ligase, transposases, chemical synthesis, etc. which technologies are known to those of skill in the art.

In certain embodiments, a selection cassette comprising an open reading frame encoding a protein that confers antibiotic resistance is positioned in between the homology arms. Host cells comprising the heterologous insert DNA inserted into their genome can be identified by culturing them on media that comprises the antibiotic to which the antibiotic resistance gene of the selection cassette provides resistance. In certain embodiments, the selection cassette may be flanked by FRT sites, which allow for later removal of the cassette by site directed recombination. Incorporating FRT sites in this manner into the donor plasmid thus ensures that the selection cassette does not remain integrated in the host cell genome. In another embodiment, the selection cassette can be removed following integration via dif site mediated site directed homologous recombination or by other, site directed chromosomal mutagenesis technologies.

The donor plasmids further can be engineered to comprise an open reading frame encoding a counterselection protein. Any gene encoding a protein known to those of skill in the art suitable for use in counterselection approaches can be incorporated into the donor plasmids described herein. In a specific embodiment, the sacB gene is used for counterselection.

The donor plasmids further can be engineered to comprise an origin of replication. Those of skill in the art will readily appreciate that the origin of replication incorporated into the donor plasmid should be suitable for use in the host cell that is undergoing genome modification. For example, an *E*. *coli* replication origin must be present when cloning is being performed in *E*. *coli.* In a specific embodiment, the origin of replication is oriT. Those of skill in the art will readily appreciate that shuttle plasmids (i.e., plasmids capable of replication in multiple host cells, e.g., multiple bacterial species) can be generated using methods known in the art, and such plasmids could be used for insertion into numerous types of host cells, e.g., prokaryotic cells, archeal cells, eubacterial cells, or eukaryotic cells. Such shuttle plasmids may comprise organism specific expression control elements and replication origins.

Step 2: A helper plasmid is made. The helper plasmid is engineered to encode all necessary activities for mediating DNA insertion into host cells and for maintenance of the helper plasmid within the host cells that undergo recombination. In certain embodiments, the helper plasmids comprise (i) a selection cassette for plasmid maintenance in the host cell, (ii) a regulon for the expression of a recombinase, i.e. an enzyme or enzymes that support and enhance the crossing over efficiency between homologous DNA stretches, (iii) a regulon for expression of a function that linearizes the DNA insert resulting in terminal homologous sequences which can undergo homologous recombination, (iv) a regulon expressing a RecA homolog for host cells that do not have an own *recA* copy and (v) a conditional origin of replication.

In certain embodiments, the helper plasmids comprise components similar to the helper plasmid pTKRED (Gene bank GU327533.1). In a specific embodiment, the helper plasmid pTKRED (Gene bank GU327533.1) is used in the method.

Step 3: The donor plasmid and the helper plasmid are introduced into the same host cell. Insertion of donor and helper plasmids can be performed by many different technologies known to those of skill in the art including, without limitation, electroporation, use of chemically competent cells, heat shock, and phage transduction. The host cells can then be cultured under selective conditions to enrich for cells carrying the introduced plasmids.

Step 4: The insertion procedure is initiated. An exemplary insertion procedure comprises the following steps: overnight cultures of positive clones (i.e. host cells comprising both the helper and donor plasmids) can be grown at, e.g., 30°C in media comprising the proper antibiotics for selection (such antibiotics can readily be selected by those of skill in the art based on the selection cassettes present in the donor/helper plasmids). The cultures then can be diluted and grown at, e.g., 30°C until exponential phase in the presence of appropriate antibiotics. Under these conditions, the helper and donor plasmids are maintained but silent. Next, the media is replaced by media containing the antibiotics for selection, as well as any inducers of conditional elements (e.g., inducible promoters or conditional origins of replication) present in the plasmids, followed by further incubation of the cells. During this time, the restriction endonuclease (e.g., *Sce*I) in the helper plasmid and the recombinase (e.g., lambda red recombinase) in the helper plasmid are expressed, leading to cleavage of the donor plasmid at the homology arms, and homologous recombination of the homology DNA at the homologous sites in the genome of the host cell. Next, the cells are plated on medium containing the component that the counterselection marker of the donor plasmid corresponds to (e.g., sucrose if the counterselection marker is sacB). This step results in counterselection of cells that comprise the donor plasmid, i.e., cells that the donor plasmid exists in an uninserted state. Such medium also comprises the resistance marker present in the insertion cassette of the donor plasmid (i.e., the antibiotic resistance cassette that is present between the HR of the donor plasmid, to select for cells that contain the heterologous insert DNA. After overnight incubation, the cells are then screened for recombined clones showing an antibiotic resistance phenotype consistent with (i) loss of the helper and donor plasmids and (ii) presence of the heterologous DNA insert.

In addition to the insertion methods described above, DNA can be inserted into the genome of a host cell using other approaches. In certain embodiments, DNA is inserted into the genome of a host cell using any site-specific insertion method known in the art. In certain embodiments, DNA is inserted into the genome of a host cell using any random integration method known in the art. Such methods are described in greater detail below.

In certain embodiments, DNA is inserted into a host cell (e.g., *E*. *coli*) genome using a method that comprises transposon-mediated insertion. Such random insertion allows for insertion of DNA of interest at multiple locations of the host cell genome, and thus allows for the identification of optimal insertion sites in host cells into which DNA has been inserted, e.g., host cells bearing inserted DNA can be compared with one another with regard to efficiency of production of the inserted DNA and host cells with highest efficiency can be selected for future use. Methods of transposon-mediated insertion of nucleic acid sequences into host cell genomes are known in the art. For example, in certain embodiments, the pUTminiTn5 delivery system (Biomedical; Sevilla, Spain) is used to stably inserted genes into the genomes of host cells (such as bacterial host cells). Strains into which DNA has been inserted then can be identified and isolated. See also Herrero et al., 1990, J. Bacteriology 172(11):6557-6567 and DeLorenzo et al., 1990, J. Bacteriology 172(11):6568-6572. In addition, in certain embodiments, transposon-mediated insertion of DNA into a host cell genome is accomplished using a Tn-7 based method of DNA insertion. See McKenzie et al., 2006, BMC Microbiology 6:39 and Sibley et al., 2012, Nucleic Acids Res. 40:e19.

In certain embodiments, DNA is inserted into a host cell (e.g., *E*. *coli*) genome using the StabyCloning™ kit or the StabyCodon T7 kit (Delphi Genetics, Charleroi, Belgium), which allow for site-specific DNA cloning.

In certain embodiments, DNA is inserted into a host cell (e.g., *E*. *coli*) genome using the "clonetegration" method of cloning and chromosomal integration of DNA. See St. Pierre et al, 2013, ACS Synthetic Biology 2:537-541.

In certain embodiments, DNA is inserted into a host cell (e.g., *E*. *coli*) genome using a method that involves conditional-replication, integration, and modular (CRIM) plasmids, as described by Haldimann and Wanner, 2001, J. Bacteriology 183:6384-6393.

In certain embodiments, DNA is inserted into a host cell (e.g., *E*. *coli*) genome using recombineering, a method described by, for example, Sharan et al., 2009, Nat. Protoc. 4:206-223; Yu et al., 2000, PNAS USA 97:5978-5983; Kuhlman et al., 2010, Nucleic Acids Res. 38:e92; and Zhang et al., 1998, Nat. Genet. 20:123-128.

In certain embodiments, heterologous nucleic acids are introduced into the modified host cells described herein by electroporation, chemical transformation by heat shock, natural transformation, phage transduction, and/or conjugation. In specific embodiments, heterologous nucleic acids are introduced into the host cells described herein using a plasmid, e.g., the heterologous nucleic acids are expressed in the host cells by a plasmid (e.g., an expression vector), and the plasmid is introduced into the modified host cells by electroporation, chemical transformation by heat shock, natural transformation, phage transduction, or conjugation.

In a specific embodiment, an isolated nucleic acid sequence is integrated into a host cell (e.g., *E*. *coli*), wherein said nucleic acid encodes a modified *Pseudomonas rfb* cluster, e.g., *Pseudomonas aeruginosa* serotype O6 *rfb* cluster, wherein said modified *Pseudomonas rfb* cluster comprises (i) a gene encoding a formyltransferase (e.g., a gene encoding SEQ ID NO:2 or a homolog thereof), (ii) a gene encoding a wzy polymerase (e.g., a gene encoding SEQ ID NO:3 or a homolog thereof); or (iii) a gene encoding a formyltransferase (e.g., a gene encoding SEQ ID NO:2 or a homolog thereof) and a gene encoding a wzy polymerase (e.g., a gene encoding SEQ ID NO:3 or a homolog thereof).

### 5.3 Bioconjugates

The modified host cells described herein can be used to produce bioconjugates comprising a *Pseudomonas* antigen linked to a carrier protein. Methods of producing bioconjugates using host cells are known in the art. See, e.g., WO 2003/074687 and WO 2006/119987. Bioconjugates, as described herein, have advantageous properties over chemical conjugates of antigen-carrier protein, in that they require less chemicals in manufacture and are more consistent in terms of the final product generated.

In a specific embodiment, provided herein is a bioconjugate comprising a carrier protein linked to a *Pseudomonas* antigen. In a specific embodiment, said *Pseudomonas* antigen is an O antigen of *Pseudomonas aeruginosa.* In a specific embodiment, provided herein is a bioconjugate comprising a *P. aeruginosa* O antigen and a carrier protein, wherein said carrier protein is EPA, PcrV (aka LcrV,EspA, SseB), PopB (YopB, YopD, FliC), or OprF, Oprl.

In a specific embodiment, provided herein is a bioconjugate comprising a carrier protein linked to a *Pseudomonas aeruginosa* O antigen, wherein said *Pseudomonas aeruginosa* O antigen is an O antigen from *Pseudomonas aeruginosa* serotype 01, O2, O3, O4, O5, O6, O7, O8, O9, 010, O11, 012, 013, 014, 015, 016, 017, 018, 019, or O20.

In a specific embodiment, provided herein is a bioconjugate comprising a carrier protein linked to a *Pseudomonas aeruginosa* O antigen, wherein said *Pseudomonas aeruginosa* O antigen is one of the serotypes described in Knirel et al., 2006, Journal of Endotoxin Research 12(6):324-336.

In a specific embodiment, provided herein is a bioconjugate comprising a carrier protein linked to a *Pseudomonas aeruginosa* O antigen, wherein said *Pseudomonas aeruginosa* O antigen is an O antigen from *Pseudomonas aeruginosa* serotype O6.

In a specific embodiment, provided herein is a bioconjugate comprising a carrier protein linked to a *Pseudomonas aeruginosa* O antigen, wherein said *Pseudomonas aeruginosa* O antigen is an O antigen from *Pseudomonas aeruginosa* serotype O11. In a specific embodiment, said O antigen from *Pseudomonas aeruginosa* serotype 011 is from *Pseudomonas aeruginosa* strain PA103 (see, e.g., Genbank Accession No. KF364633.1).

The bioconjugates described herein can be purified by any method known in the art for purification of a protein, for example, by chromatography (e.g., ion exchange, anionic exchange, affinity, and sizing column chromatography), centrifugation, differential solubility, or by any other standard technique for the purification of proteins. See, e.g., Saraswat et al., 2013, Biomed. Res. Int. ID#312709 (p. 1-18); see also the methods described in WO 2009/104074. Further, the bioconjugates may be fused to heterologous polypeptide sequences described herein or otherwise known in the art to facilitate purification. The actual conditions used to purify a particular bioconjugate will depend, in part, on the synthesis strategy and on factors such as net charge, hydrophobicity, and/or hydrophilicity of the bioconjugate, and will be apparent to those having skill in the art.

### 5.4 Analytical Methods

Various methods can be used to analyze the structural compositions and sugar chain lengths of the bioconjugates described herein.

In one embodiment, hydrazinolysis can be used to analyze glycans. First, polysaccharides are released from their protein carriers by incubation with hydrazine according to the manufacturer's instructions (Ludger Liberate Hydrazinolysis Glycan Release Kit, Oxfordshire, UK). The nucleophile hydrazine attacks the glycosidic bond between the polysaccharide and the carrier protein and allows release of the attached glycans. N-acetyl groups are lost during this treatment and have to be reconstituted by re-N-acetylation. The free glycans are purified on carbon columns and subsequently labeled at the reducing end with the fluorophor 2-amino benzamide. See Bigge JC, Patel TP, Bruce JA, Goulding PN, Charles SM, Parekh RB: Nonselective and efficient fluorescent labeling of glycans using 2-amino benzamide and anthranilic acid. Anal Biochem 1995, 230(2):229-238. The labeled polysaccharides are separated on a GlycoSep-N column (GL Sciences) according to the HPLC protocol of Royle *et al..* See Royle L, Mattu TS, Hart E, Langridge JI, Merry AH, Murphy N, Harvey DJ, Dwek RA, Rudd PM: An analytical and structural database provides a strategy for sequencing O-glycans from microgram quantities of glycoproteins. Anal Biochem 2002, 304(1):70-90. The resulting fluorescence chromatogram indicates the polysaccharide length and number of repeating units. Structural information can be gathered by collecting individual peaks and subsequently performing MS/MS analysis. Thereby the monosaccharide composition and sequence of the repeating unit could be confirmed and additionally in homogeneity of the polysaccharide composition could be identified.

In another embodiment, SDS-PAGE or capillary gel electrophoresis can be used to assess glycans and bioconjugates. Polymer length for the O antigen glycans is defined by the number of repeat units that are linearly assembled. This means that the typical ladder like pattern is a consequence of different repeat unit numbers that compose the glycan. Thus, two bands next to each other in SDS PAGE or other techniques that separate by size differ by only a single repeat unit. These discrete differences are exploited when analyzing glycoproteins for glycan size: The unglycosylated carrier protein and the bioconjugate with different polymer chain lengths separate according to their electrophoretic mobilities. The first detectable repeating unit number (n₁) and the average repeating unit number (n_{average}) present on a bioconjugate are measured. These parameters can be used to demonstrate batch to batch consistency or polysaccharide stability.

In another embodiment, high mass MS and size exclusion HPLC could be applied to measure the size of the complete bioconjugates.

In another embodiment, an anthrone-sulfuric acid assay can be used to measure polysaccharide yields. See Leyva A, Quintana A, Sanchez M, Rodriguez EN, Cremata J, Sanchez JC: Rapid and sensitive anthrone-sulfuric acid assay in microplate format to quantify carbohydrate in biopharmaceutical products: method development and validation. Biologicals : journal of the International Association of Biological Standardization 2008, 36(2):134-141. In another embodiment, a Methylpentose assay can be used to measure polysaccharide yields. See, e.g., Dische et al., J Biol Chem. 1948 Sep;175(2):595-603.

### Change in glycosylation site usage

To show that the site usage in a specific protein is changed in a multiple plasmid system as opposed to an inserted system, the glycosylation site usage must be quantified. Methods to do so are listed below.

Glycopeptide LC-MS/MS: bioconjugates are digested with protease(s), and the peptides are separated by a suitable chromatographic method (C18, Hydrophilic interaction HPLC HILIC, GlycoSepN columns, SE HPLC, AE HPLC), and the different peptides are identified using MS/MS. This method can be used with our without previous sugar chain shortening by chemical (smith degradation) or enzymatic methods. Quantification of glycopeptide peaks using UV detection at 215 to 280 nm allow relative determination of glycosylation site usage.

Size exclusion HPLC: Higher glycosylation site usage is reflected by a earlier elution time from a SE HPLC column.

### Homogeneity

Bioconjugate homogeneity (i.e., the homogeneity of the attached sugar residues) can be assessed using methods that measure glycan length and hydrodynamic radius.

### Other Potential Clinical/Practical Applications

Integrated strains can make a higher yield of bioconjugates due to the reduced antibiotic selection burden as compared to the three plasmid system. In addition, less proteolytic degradation occurs due to reduced metabolic burden to the cells.

Integrated strains make bioconjugates with shorter, less spread polysaccharide length distributions. Thus, the bioconjugates are easier to characterize and are better defined. In addition, insertion may reduce the extent of periplasmic stress to the cells which may lead to less proteolysis of product during the fermentation process due to the reduced antibiotic selection burden as compared to the three plasmid system.

Protein glycosylation systems require three recombinant elements in the production host: a carrier protein expression DNA, an oligosaccharyl transferase expression DNA, and a polysaccharide expression DNA. Prior art bacterial production systems contain these three elements on plasmids. Thus, there is a risk for instability during manufacture due to plasmid loss, particularly because antibiotics used for maintenance of the plasmids mustn't be present during fermentation of GMP material. Since inserted strains contain one plasmid less, they are more stable over many generations. This means that higher scale fermentations and longer incubation times (higher generation numbers) are more feasible. In addition, the absence of an antibiotic for selection makes a safer product, due to the absence of trace antibiotics which can cause allergic reactions in sensitive subjects. See COMMITTEE WE, BIOLOGICAL O, STANDARDIZATION: WHO Technical Report Series 941. In: Fifty-sixth Report. Edited by Organization WH. Geneva: World Health Organization; 2007.

Inserted strains are more genetically stable due to the fixed chromosomal insertion, thus leading to higher reproducibility of desired protein products during the production process, e.g., during culture of host cell comprising inserted heterologous DNA.

### Analytical Methods for Testing Benefit

*Yield.* Yield is measured as carbohydrate amount derived from a liter of bacterial production culture grown in a bioreactor under controlled and optimized conditions. After purification of bioconjugate, the carbohydrate yields can be directly measured by either the anthrone assay or ELISA using carbohydrate specific antisera. Indirect measurements are possible by using the protein amount (measured by well known BCA, Lowry, or bardford assays) and the glycan length and structure to calculate a theoretical carbohydrate amount per gram of protein. In addition, yield can also be measured by drying the glycoprotein preparation from a volatile buffer and using a balance to measure the weight.

*Homogeneity.* Homogeneity means the variability of glycan length and possibly the number of glycosylation sites. Methods listed above can be used for this purpose. SE-HPLC allows the measurement of the hydrodynamic radius. Higher numbers of glycosylation sites in the carrier lead to higher variation in hydrodynamic radius compared to a carrier with less glycosylation sites. However, when single glycan chains are analyzed, they may be more homogenous due to the more controlled length. Glycan length is measured by hydrazinolysis, SDS PAGE, and CGE. In addition, homogeneity can also mean that certain glycosylation site usage patterns change to a broader/narrower range. These factors can be measured by Glycopeptide LC-MS/MS.

*Strain stability and reproducibility.* Strain stability during bacterial fermentation in absence of selective pressure is measured by direct and indirect methods that confirm presence or absence of the recombinant DNA in production culture cells. Culture volume influence can be simulated by elongated culturing times meaning increased generation times. The more generations in fermentation, the more it is likely that a recombinant element is lost. Loss of a recombinant element is considered instability. Indirect methods rely on the association of selection cassettes with recombinant DNA, e.g. the antibiotic resistance cassettes in a plasmid. Production culture cells are plated on selective media, e.g. LB plates supplemented with antibiotics or other chemicals related to a selection system, and resistant colonies are considered as positive for the recombinant DNA associated to the respective selection chemical. In the case of a multiple plasmid system, resistant colonies to multiple antibiotics are counted and the proportion of cells containing all three resistances is considered the stable population. Alternatively, quantitative PCR can be used to measure the amount of recombinant DNA of the three recombinant elements in the presence, absence of selection, and at different time points of fermentation. Thus, the relative and absolute amount of recombinant DNA is measured and compared. Reproducibility of the production process is measured by the complete analysis of consistency batches by the methods stated in this application.

### 5.5 Compositions

### Compositions Comprising Host Cells

In one aspect, provided herein are compositions comprising the host cells described herein (see Section 5.1). Such compositions can be used in methods for generating the bioconjugates described herein (see Section 5.3), e.g., the compositions comprising host cells can be cultured under conditions suitable for the production of proteins. Subsequently, bioconjugates can be isolated from said compositions comprising host cells using methods known in the art.

The compositions comprising the host cells provided herein can comprise additional components suitable for maintenance and survival of the host cells described herein, and can additionally comprise additional components required or beneficial to the production of proteins by the host cells, e.g., inducers for inducible promoters, such as arabinose, IPTG.

### Compositions Comprising Bioconjugates

In another aspect, provided herein are compositions (e.g., pharmaceutical compositions) comprising one or more of the bioconjugates described herein (see Section 5.3). The compositions described herein are useful in the treatment and prevention of infection of subjects (e.g., human subjects) by *Pseudomonas.* See Section 5.6.

In certain embodiments, in addition to comprising a bioconjugate described herein (see Section 5.3), the compositions (e.g., pharmaceutical compositions) described herein comprise a pharmaceutically acceptable carrier. As used herein, the term "pharmaceutically acceptable" means approved by a regulatory agency of the Federal or a state government or listed in the U.S. Pharmacopeia or other generally recognized pharmacopeiae for use in animals, and more particularly in humans. The term "carrier," as used herein in the context of a pharmaceutically acceptable carrier, refers to a diluent, adjuvant, excipient, or vehicle with which the pharmaceutical composition is administered. Saline solutions and aqueous dextrose and glycerol solutions can also be employed as liquid carriers, particularly for injectable solutions. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Examples of suitable pharmaceutical carriers are described in "Remington's Pharmaceutical Sciences" by E.W. Martin.

In a specific embodiment, provided herein is a composition (e.g., pharmaceutical composition) comprising a bioconjugate comprising a carrier protein linked to a *Pseudomonas* antigen. In a specific embodiment, provided herein is a composition (e.g., pharmaceutical composition) comprising a bioconjugate comprising a carrier protein linked to a an O antigen of *Pseudomonas aeruginosa.*

In a specific embodiment, provided herein is a composition (e.g., pharmaceutical composition) comprising a bioconjugate comprising a carrier protein linked to a *Pseudomonas aeruginosa* O antigen, wherein said *Pseudomonas aeruginosa* O antigen is an O antigen from *Pseudomonas aeruginosa* serotype O1, O2, O3, O4, O5, O6, O7, O8, O9, 010, O11, 012, 013, 014, 015, 016, 017, 018, 019, or O20.

In a specific embodiment, provided herein is a composition (e.g., pharmaceutical composition) comprising a bioconjugate comprising a carrier protein linked to a *Pseudomonas aeruginosa* O antigen, wherein said *Pseudomonas aeruginosa* O antigen is an O antigen from *Pseudomonas aeruginosa* serotype O6.

In a specific embodiment, provided herein is a composition (e.g., pharmaceutical composition) comprising a bioconjugate comprising a carrier protein linked to a *Pseudomonas aeruginosa* O antigen, wherein said *Pseudomonas aeruginosa* O antigen is an O antigen from *Pseudomonas aeruginosa* serotype O11. In a specific embodiment, said O antigen from *Pseudomonas aeruginosa* serotype O11 is from *Pseudomonas aeruginosa* strain PA103 (see, e.g., Genbank Accession No. KF364633.1).

The compositions comprising bioconjugates that are provided herein can be used for eliciting an immune response in a host to whom the composition is administered, i.e., the compositions are immunogenic. Thus, the compositions described herein can be used as vaccines against *Pseudomonas* infection, or can be used in the treatment of *Pseudomonas* infection.

The compositions comprising the bioconjugates described herein may comprise any additional components suitable for use in pharmaceutical administration. In specific embodiments, the compositions described herein are monovalent formulations. In other embodiments, the compositions described herein are multivalent formulations, e.g., bivalent, trivalent, and tetravalent formulations. For example, a multivalent formulation comprises more than one bioconjugate described herein.

In certain embodiments, the compositions described herein additionally comprise a preservative, e.g., the mercury derivative thimerosal. In a specific embodiment, the pharmaceutical compositions described herein comprise 0.001% to 0.01% thimerosal. In other embodiments, the pharmaceutical compositions described herein do not comprise a preservative.

In certain embodiments, the compositions described herein (e.g., the immunogenic compositions) comprise, or are administered in combination with, an adjuvant. The adjuvant for administration in combination with a composition described herein may be administered before, concomitantly with, or after administration of said composition. In some embodiments, the term "adjuvant" refers to a compound that when administered in conjunction with or as part of a composition described herein augments, enhances and/or boosts the immune response to a bioconjugate, but when the compound is administered alone does not generate an immune response to the bioconjugate. In some embodiments, the adjuvant generates an immune response to the poly bioconjugate peptide and does not produce an allergy or other adverse reaction. Adjuvants can enhance an immune response by several mechanisms including, e.g., lymphocyte recruitment, stimulation of B and/or T cells, and stimulation of macrophages.

Specific examples of adjuvants include, but are not limited to, aluminum salts (alum) (such as aluminum hydroxide, aluminum phosphate, and aluminum sulfate), 3 De-O-acylated monophosphoryl lipid A (MPL) (see United Kingdom Patent GB2220211), MF59 (Novartis), AS03 (GlaxoSmithKline), AS04 (GlaxoSmithKline), polysorbate 80 (Tween 80; ICL Americas, Inc.), imidazopyridine compounds (see International Application No. PCT/US2007/064857, published as International Publication No. WO2007/109812), imidazoquinoxaline compounds (see International Application No. PCT/US2007/064858, published as International Publication No. WO2007/109813) and saponins, such as QS21 (see Kensil et al., in Vaccine Design: The Subunit and Adjuvant Approach (eds. Powell & Newman, Plenum Press, NY, 1995); U.S. Pat. No. 5,057,540). In some embodiments, the adjuvant is Freund's adjuvant (complete or incomplete). Other adjuvants are oil in water emulsions (such as squalene or peanut oil), optionally in combination with immune stimulants, such as monophosphoryl lipid A (see Stoute et al., N. Engl. J. Med. 336, 86-91 (1997)). Another adjuvant is CpG (Bioworld Today, Nov. 15, 1998).

In certain embodiments, the compositions described herein are formulated to be suitable for the intended route of administration to a subject. For example, the compositions described herein may be formulated to be suitable for subcutaneous, parenteral, oral, intradermal, transdermal, colorectal, intraperitoneal, and rectal administration. In a specific embodiment, the pharmaceutical composition may be formulated for intravenous, oral, intraperitoneal, intranasal, intratracheal, subcutaneous, intramuscular, topical, intradermal, transdermal or pulmonary administration.

In certain embodiments, the compositions described herein additionally comprise one or more buffers, *e.g.,* phosphate buffer and sucrose phosphate glutamate buffer. In other embodiments, the compositions described herein do not comprise buffers.

In certain embodiments, the compositions described herein additionally comprise one or more salts, *e.g.,* sodium chloride, calcium chloride, sodium phosphate, monosodium glutamate, and aluminum salts (*e.g.*, aluminum hydroxide, aluminum phosphate, alum (potassium aluminum sulfate), or a mixture of such aluminum salts). In other embodiments, the compositions described herein do not comprise salts.

The compositions described herein can be included in a container, pack, or dispenser together with instructions for administration.

The compositions described herein can be stored before use, *e.g.,* the compositions can be stored frozen (*e.g.,* at about -20°C or at about -70°C); stored in refrigerated conditions (*e.g*., at about 4°C); or stored at room temperature.

### 5.6 Prophylactic and Therapeutic Uses

In one aspect, provided herein are methods of treating a *Pseudomonas* infection in a subject comprising administering to the subject a bioconjugate described herein (see Section 5.3) or a composition thereof (see Section 5.5). In another aspect, provided herein are methods of preventing a *Pseudomonas* infection in a subject comprising administering to the subject a bioconjugate described herein (see Section 5.3) or a composition thereof (see Section 5.5).

Also provided herein are methods of inducing an immune response in a subject against *Pseudomonas,* comprising administering to the subject a bioconjugate described herein (see Section 5.3) or a composition described herein (see Section 5.5). In one embodiment, said subject has a *Pseudomonas* infection at the time of administration. In another embodiment, said subject does not have a *Pseudomonas* infection at the time of administration.

In a specific embodiment, provided herein is a method for preventing a *Pseudomonas* infection in a subject, wherein said method comprises administering to a subject in need thereof an effective amount of a composition described in Section 5.5. The methods of preventing a *Pseudomonas* infection in a subject provided herein result in the induction of an immune response in a subject comprising administering to the subject a of a composition described in Section 5.5. One of skill in the art will understand that the methods of inducing an immune response in a subject described herein result in vaccination of the subject against infection by *Pseudomonas* strains whose antigens are present in the composition(s).

In a specific embodiment, provided herein is a method for treating a *Pseudomonas* infection in a subject, wherein said method comprises administering to a subject in need thereof an effective amount of a composition described in Section 5.5.

In certain embodiments, the immune response induced by a bioconjugate described herein (see Section 5.3) or a composition described herein (see Section 5.5) is effective to prevent and/or treat a *Pseudomonas* infection caused by *Pseudomonas aeruginosa.* In certain embodiments, the immune response induced by a bioconjugate described herein (see Section 5.3) or a composition described herein (see Section 5.5) is effective to prevent and/or treat a *Pseudomonas* infection by more than one strain or serotype of *Pseudomonas aeruginosa.*

In a specific embodiment, the immune response induced by a bioconjugate described herein (see Section 5.3) or a composition described herein (see Section 5.5) is effective to prevent and/or treat an infection caused by *Pseudomonas aeruginosa* serotype O1, O2, O3, O4, O5, O6, O7, O8, O9, 010, O11, 012, 013, 014, 015, 016, 017, 018, 019, or O20. In another specific embodiment, said *rfb* cluster from *Pseudomonas aeruginosa* is the *rfb* cluster from any one of the serotypes described in Knirel et al., 2006, Journal of Endotoxin Research 12(6):324-336. In a specific embodiment, the immune response induced by a bioconjugate described herein (see Section 5.3) or a composition described herein (see Section 5.5) is effective to prevent and/or treat an infection caused by *Pseudomonas aeruginosa* serotype O6. In a specific embodiment, the immune response induced by a bioconjugate described herein (see Section 5.3) or a composition described herein (see Section 5.5) is effective to prevent and/or treat an infection caused by *Pseudomonas aeruginosa* serotype O11. In a specific embodiment, said *Pseudomonas aeruginosa* serotype 011 is *Pseudomonas aeruginosa* strain PA103 (see, e.g., Genbank Accession No. KF364633.1).

In certain embodiments, the immune response induced in a subject following administration of a bioconjugate described herein (see Section 5.3) or a composition described herein (see Section 5.5) is effective to reduce one or more symptoms resulting from a *Pseudomonas* infection.

In certain embodiments, the immune response induced in a subject following administration of a bioconjugate described herein (see Section 5.3) or a composition described herein (see Section 5.5) is effective to reduce the likelihood of hospitalization of a subject suffering from a *Pseudomonas* infection. In some embodiments, the immune response induced in a subject following administration of a bioconjugate described herein (see Section 5.3) or a composition described herein (see Section 5.5) is effective to reduce the duration of hospitalization of a subject suffering from a *Pseudomonas* infection.

### 5.7 Assays

### Assay for Assessing Ability of Bioconjugates to Induce an Immune Response

The ability of the bioconjugates/compositions described herein to generate an immune response in a subject can be assessed using any approach known to those of skill in the art or described herein. In some embodiments, the ability of a bioconjugate to generate an immune response in a subject can be assessed by immunizing a subject (*e.g.,* a mouse) or set of subjects with a bioconjugate described herein and immunizing an additional subject (*e.g.,* a mouse) or set of subjects with a control (PBS). The subjects or set of subjects can subsequently be challenged with *Pseudomonas* and the ability of the *Pseudomonas* to cause disease in the subjects or set of subjects can be determined. Those skilled in the art will recognize that if the subject or set of subjects immunized with the control suffer(s) from disease subsequent to challenge with the *Pseudomonas* but the subject or set of subjects immunized with a bioconjugate(s) or composition thereof described herein suffers less from or do not suffer from disease, then the bioconjugate is able to generate an immune response in a subject. The ability of a bioconjugate(s) or composition thereof described herein to induce antiserum that cross-reacts with a *Pseudomonas* antigen can be tested by, e.g., an immunoassay, such as an ELISA.

### In Vitro Bactericidal Assays

The ability of the bioconjugates described herein to generate an immune response in a subject can be assessed using a serum bactericidal assay (SBA) or opsonophagocytotic killing assay (OPK), which represents an established and accepted method that has been used to obtain approval of glycoconjugate-based vaccines. Such assays are well-known in the art and, briefly, comprise the steps of generating and isolating antibodies against a target of interest (e.g., an antigen of *Pseudomonas*) by administering to a subject (e.g., a mouse) a compound that elicits such antibodies. Subsequently, the bactericidal capacity of the antibodies can be assessed by, e.g., culturing the bacteria in question in the presence of said antibodies and complement and - depending on the assay - neutrophilic cells and assaying the ability of the antibodies to kill and/or neutralize the bacteria, e.g., using standard microbiological approaches.

### 6. EXAMPLES

### Example 1: Bacterial Strains with an Inserted Oligosaccharyl Transferase and an Inserted rfb Cluster Are Stable and Produce Bioconjugates

This example demonstrates that bioconjugates can successfully be produced by a bacterial host strain that has been genetically modified by insertion of (i) a nucleic acid encoding an oligosaccharyl transferase and (ii) a nucleic acid encoding an *rfb* cluster.

Modified *E*. *coli* host cells were generated by inserting the following directly into the host cell genome: (i) a nucleic acid encoding the *C. jejuni* oligosaccharyl transferase (PglB) and (ii) a nucleic acid encoding the *rfb* cluster from *Pseudomonas aeruginosa* strain PA103. This *rfb* cluster encodes genes necessary for O-antigen synthesis of the *Pseudomonas aeruginosa* serogroup O11 antigen. The insertions were performed using the novel insertion method described in PCT/EP2013/071328 (see Section 5.2, above) or the pUT mini system (Biomedal Lifescience). The insertion method described in PCT/EP2013/071328 is site-specific and utilizes homologous recombination, whereas the pUT mini system is a random, transposon-mediated approach that results in a nucleic acid sequence of interest being randomly inserted into a host cell genome. The *E*. *coli* host cells further were modified by introduction of a plasmid that expresses detoxified *Pseudomonas* extotoxin A (EPA) as a carrier protein into the host cells. Thus, the modified *E*. *coli* host cells described in this example express (i) the *C. jejuni* oligosaccharyl transferase (PglB), by virtue of integration of a nucleic acid encoding the oligosaccharyl transferase into the host cell genome; (ii) genes of a *Pseudomonas aeruginosa rfb* cluster that produce the O11 antigen, by virtue of integration of a nucleic acid encoding the *rfb* cluster from *Pseudomonas aeruginosa* strain PA103 into the host cell genome; and (iii) the EPA carrier protein, by virtue of transforming the host cell with a plasmid comprising a nucleic acid encoding the carrier protein.

Additional modified *E*. *coli* host cells were generated to allow for comparison of the ability of the modified host cells comprising double integrations (integration of an oligosaccharyl transferase and integration of an *rfb* cluster) to produce bioconjugates (EPA-O11) with bioconjugate production by host cells having (i) only a single integration of the oligosaccharyl transferase or the *rfb* cluster and the remaining components (carrier protein and oligosaccharyl transferase or *rfb* cluster) plasmid expressed by the host cell; or (ii) no integrated components, with all components (carrier protein and oligosaccharyl transferase and *rfb* cluster) plasmid expressed.

Three different *E*. *coli* background strains were used in the analysis: (i) "St4167" (W3110 Δ*waaL,* ΔrfbO16::rfbP.a.O11), which comprises a deletion of the *E*. *coli waaL* gene, a deletion of the *E*. *coli* 016 *rfb* cluster, and an insertion of the *P. aeruginosa* O11 *rfb* cluster (PCT/EP2013/071328); (ii) "St1128'' (W3110 Δ*waaL*), which comprises a deletion of the *E*. *coli waaL* gene; and (iii) "St1935" (W3110 Δ*waaL,* Δ*wzzE-wecG,* Δ*wbbIJK*), which comprises deletion of the indicated genes. For insertion of the *P. aeruginosa* O11 *rfb* cluster in St4167, O11 *rfb* cluster was cloned into the pDOC plasmid and the method according to PCT/EP2013/071328 was employed. The St4167 strains represent the double integration strains.

The specific plasmids utilized to introduce EPA into the host cell strains are designated "p1077" and "p150." The latter is described in Ihssen, et al., (2010) Microbial cell factories 9, 61, and the plasmids are the same with the exception of the fact that p1077 replaces the Amp cassette of p150 with a Kan cassette.

The following St4167 variants were generated: (i) St4167 with *pglB* inserted in place of the host cell *yahL* gene (by the method of PCT/EP2013/071328) and EPA expressed by plasmid p1077; (ii) St4167 with *pglB* inserted in place of the host cell *ompT* gene (using the pUT mini system) and EPA expressed by plasmid p150; (iii) St4167 with *pglB* expressed by plasmid p1769 (*pglB* in pDOC) and EPA expressed by plasmid p1077; (iv) St4167 with *pglB* expressed by plasmid p939 (pEXT21 based expression plasmid for PglB with an HA tag, codon optimized) and EPA expressed by plasmid p1077; and (v) St4167 with *pglB* expressed by plasmid p1762 (*pglB* in pDOC) and EPA expressed by plasmid p1077.

The following St1128 variants were generated: (i) St1128 with *pglB* expressed by plasmid p939, *P. aeruginosa* O11 *rfb* cluster expressed by plasmid p164 (pLAFR plasmid engineered to contain the *P. aeruginosa* O11 *rfb* cluster), and EPA expressed by plasmid p1077; and (ii) St1128 with *pglB* inserted in place of the host cell *yahL* gene (by the method of PCT/EP2013/071328), *P. aeruginosa* O11 *rfb* cluster expressed by plasmid p164, and EPA expressed by plasmid p1077.

The following St1935 variants were generated: (i) St1935 with *pglB* inserted in place of the host cell *ompT* gene (by the method of PCT/EP2013/071328), *P. aeruginosa* 011 *rfb* cluster expressed by plasmid p164, and EPA expressed by plasmid p1077; (ii) St1935 with *pglB* inserted in place of the host cell *yahL* gene (by the method of PCT/EP2013/071328), *P. aeruginosa* O11 *rfb* cluster expressed by plasmid p164, and EPA expressed by plasmid p1077; and St1935 with *pglB* expressed by plasmid p939, P. *aeruginosa* O11 *rfb* cluster expressed by plasmid p164, and EPA expressed by plasmid p1077.

As shown in Figure 1, all strains expressing an oligosaccharyl transferase, carrier protein, and an *rfb* cluster produced bioconjugates. See the blots depicted between kDa markers 100 and 130, which correspond to EPA-O11. Importantly, this observation includes strains comprising double integration of an oligosaccharyl transferase and an *rfb* cluster. See, in particular, the results shown for St4167. Thus, this Example demonstrates not only that stable host cells can be generated following double insertion of genes/gene clusters into the host cell genome, but that function of the genes is maintained. Specifically, function of the inserted oligosaccharyl transferase and inserted *rfb* cluster was preserved, resulting in the production of bioconjugates.

### Example 2: Identification of a formyltransferase gene that contributes to the synthesis of a native P. aeruginosa O6 O-antigen oligo/polysaccharide

This example describes the identification of the *Pseudomonas aeruginosa* O6 formyltransferase.

Proteome data for the *Pseudomonas aeruginosa* O6 strain "LESB58," the genome of which is known, was searched for domains containing homology to the prototype query domains "Formyltransferase" and "FMT C-terminal domain-like" domain using the algorithm provided at www.supfam.org/SUPERFAMILY/. The search identified 9 protein sequences with possible related domains.

To evaluate whether any of the 9 candidates identified were specific for O6 (and thereby for a formylated O antigen repreat unit) their absence in the proteome of another *Pseudomonas aeruginosa* serotype (O5, strain PAO1) was analyzed using a BLAST search (NCBI website). The *Pseudomonas aeruginosa* O5 O-antigen structure is unrelated that of *Pseudomonas aeruginosa* O6. Specifically, no formyl group is present in the O5 structure. 8 out of 9 candidates had homologues in *Pseudomonas aeruginosa* serotype O5 which indicated that these proteins were unspecific for *Pseudomonas aeruginosa* O6 strain LESB58. The remaining candidate (locus_tag=PLES_12061, GenBank: CAW25933.1; SEQ ID NO:2) had no obvious homologue in *Pseudomonas aeruginosa* serotype O5 and was therefore classified as specific for LESB58/*Pseudomonas aeruginosa* serotype O6.

To confirm O6 specificity, the presence of the discovered *Pseudomonas aeruginosa* serotype O6 formyltransferase (SEQ ID NO:2) in other *Pseudomonas aeruginosa* serotype O6 strains was verified. Proteins equivalent to the *Pseudomonas aeruginosa* serotype O6 formyltransferase (SEQ ID NO:2) were identified in four other *Pseudomonas aeruginosa* serotype O6 strains, including locus tag: PAK 01412 in strain "PAK" and locus tag: PAM18_1171 in strain M18.

Formyltransferases with low amino acid sequence identity to *Pseudomonas aeruginosa* serotype O6 formyltransferase (SEQ ID NO:2) also were identified in *Methylobacterium sp.* (33% identity, ACCESSION WP_020093860), *Thiothrix nivea* (30% identity, ACCESSION WP_002707142), *Anaerophaga thermohalophila* (28% identity, ACCESSION WP_010422313), *Halorubrum californiense* (27% identity, ACCESSION WP_008445073), *Azorhizobium caulinodans* (25% identity, ACCESSION WP_012170036) and *Burkholderia glathei* (24% identity, ACCESSION KDR39707). Taken together, these homology analyses indicated that the related genes encode an O6 specific activity related to formylation.

To test the functional activity of the *Pseudomonas aeruginosa* serotype O6 formyltransferase (SEQ ID NO:2) on the non formylated O6 repeat unit structure, the gene encoding SEQ ID NO:2 was cloned. The rare TTG START codon of the gene was replaced by ATG. A schematic representation of the cloning of the *Pseudomonas aeruginosa* serotype O6 formyltransferase (SEQ ID NO:2) into the *Pseudomonas aeruginosa* O6 rfb cluster and the relative organization of the genes is depicted in Figure 5.

Once identified, function of the *Pseudomonas aeruginosa* O6 formyltransferase was assessed. *Pseudomonas aeruginosa* serotype O6 formyltransferase (SEQ ID NO:2) was tested for functionality by co-expression with the *rfb* cluster genes of *Pseudomonas aeruginosa* O6 in *E*. *coli* strains that lack a functional ECA (wec) cluster. To show formylation, single antigen repeat units bound to lipid a core were analyzed (in a *waaL* positive strain). The formylated O6 O-antigen repeating unit was identified by immunodetection using an O6 specific antibody (Figure 3A) indicating that the formyl group is a relevant epitope of the *Pseudomonas aeruginosa* O6 O antigen structure.

To show formylation on the molecular level, O6 repeat units were analyzed by MALDI MSMS. Purified and 2AB labelled repeat units showed that coexpression of *Pseudomonas aeruginosa* serotype O6 formyltransferase (SEQ ID NO:2) with the *rfb* cluster genes of *Pseudomonas aeruginosa* O6 gave rise to a fluorescence signal of the main peak which was shifted by 2-3 minutes (from 58 to 61', Fig. 3B).

MALDI-MSMS analysis of the material contained in the peaks at 58' resulted in a Y ion fragmentation series which is in agreement with the non formylated, N acetylated 2-AB labelled O6 repeat unit. The protonated precursor ion m/z=905, fragmented into a prominent ion series of 905->759->543->326, corresponding to losses of 146 (deoxyhexose), 216 (amidated N-acetylhexosaminuronic acid), 217 (N-acetylhexosaminuronic acid) units. Material collected at 61' obtained from cells expressing the *Pseudomonas aeruginosa* serotype O6 formyltransferase gene contained a prominent precursor ion of 891, which fragmented at 891->745->529->326, corresponding to losses of 146 (as above), 216 (as above), and 203 (amidated N-formylhexosaminuronic acid). This data proved that formylation is dependent on the expression of *Pseudomonas aeruginosa* serotype O6 formyltransferase and that accordingly the gene is encoding the formyltransferase. Thus, the gene that encodes the *Pseudomonas aeruginosa* serotype O6 formyltransferase was named fmtO6. The fact that the acetyl group of the amidated N-acetylhexosaminuronic acid is replaced by a formyl group suggests a two step mechanism wherein the acetyl group is first removed before the formyl group can be added. This model implies that a free amine group would be present at C2 as an intermediate before the formyltransferase domain attaches a formyl group to the monosaccharide. Thus, deacetylated and non formylated O antigen may be a substantial and immunologically relevant, substochiometrically present polysaccharide form of *P. aeruginosa* serotype O6.

### Example 3: Identification and testing of the wzy gene for polymerization of the P. aeruginosa O6 O antigen.

This example describes the identification of the *Pseudomonas aeruginosa* O6 *wzy* polymerase.

O antigen polysaccharides constitute the outer cell surface of many Gram negative bacteria. The enzymatic machinery responsible for the biosynthesis of O antigen is often encoded in a single gene cluster called the *rfb* cluster. *Pseudomonas aeruginosa* serotype O6 strains express a polymeric O-antigen (Figure 2). However, in the respective O-antigen cluster, a gene encoding an O antigen polymerase (wzy) is absent. This means that in order to recombinantly express the *P. aeruginosa* O6 O antigen in *E. coli,* identification of the wzy gene was necessary. O-antigen polymerases (wzy) are integral inner membrane proteins that catalyze the polymerization of O-antigen repeating units in the periplasmic space before "en bloc" ligation to the lipid A-core Oligosaccharide to form LPS. Wzy polymerases are highly specific for their repeat unit oligomer and homologies among *wzy* genes are poor.

The O-antigen of *Pseudomonas aeruginosa* 019 shares structural similarities to that of *Pseudomonas aeruginosa* O6. It was speculated that the *wzy* proteins that recognize both structures might also share similar properties, e.g., structure, sequence, number of transmembrane domains. The sequence of the O19 *Wzy* protein of *Pseudomonas aeruginosa* 019 (ACCESSION AAM27560) is known and was used as a primary query in a Blast analysis using the *Pseudomonas aeruginosa* O6 PAK strain proteome as the subject for the homology search.

To evaluate whether the candidates identified were specific for *Pseudomonas aeruginosa* O6, their presence in the proteome of another *Pseudomonas aeruginosa* serotype (O5, strain PAO1) was analyzed. The O5 O-antigen structure is unrelated to that of O6 and 019. The Top 100 results were analyzed individually for the presence in the *Pseudomonas aeruginosa* O5 proteome using blast analysis. 97 out of 100 candidates from the PAK proteome had homologues in the *Pseudomonas aeruginosa* serotype O5 proteome which indicated that these proteins were generally present in *Pseudomonas aeruginosa* strains and possibly unrelated to O6 O antigen biosynthesis. Three out of the 100 candidates had no obvious homologue in the *Pseudomonas aeruginosa* O5 proteome, and were therefore determined to be *Pseudomonas aeruginosa* O6 specific.

To test whether one of the three identified candidate proteins was a *Pseudomonas aeruginosa* O6 *wzy,* the three proteins were used as query in a Blast analysis. One of the three candidates, PAK_01823 (O6wzy PAK 01823; SEQ ID NO:3), shared amino acid sequence identity to other, known oligosaccharide repeat unit polymerases, e.g, 25% identity to *Streptococcus sanguinis* oligosaccharide repeat unit polymerases (ACCESSION WP_004192559) and 22% identity to *Escherichia coli* 0139 oligosaccharide repeat unit polymerases (ACCESSION AAZ85718). Thus, PAK_01823 (06wzy PAK_01823; SEQ ID NO:3) was identified as the *Pseudomonas aeruginosa* O6 *wzy.*

To further confirm SEQ ID NO:3 as the protein encoded by the *Pseudomonas aeruginosa* O6 *wzy,* the subcellular localization of the protein was predicted bioinformatically using PSORTb (www.psort.org/psortb/). The protein was predicted to be localized in the cytoplasmic membrane with 11 transmembrane domains, a feature that is common among O-antigen polymerases.

Proteins equivalent to PAK_01823 (O6wzy PAK_01823; SEQ ID NO:3) were found in other O6 positive *P. aeruginosa* strains, including the LESB58 strain (which had a *Pseudomonas aeruginosa* O6 wzy protein with only 1 aa difference compared to the PAK strain and a strain tested internally).

Next, functional testing of the *Pseudomonas aeruginosa* O6 *wzy* was carried out. The *Pseudomonas aeruginosa* O6 rfb cluster, the *fmtO6* gene (i.e., the gene encoding SEQ ID NO:2, discussed in Example 2, above), and the gene encoding *Pseudomonas aeruginosa* O6 *wzy* (i.e., the gene encoding SEQ ID NO:3) were co-expressed in *E*. *coli* W3110 Δwec cells, and the lipopolysaccharide formed was analyzed by immunoblotting (Fig. 4). Anti-O6 antiserum detected a ladder like signal only in the sample originating from the cells that contained all three transgenes, indicating that PAK_01823 (O6wzy PAK_01823; SEQ ID NO:3) is indeed the polymerase of *P. aeruginosa* O6. Thus, the gene encoding PAK_01823 was named *O6wzy.*

To generate a single gene cluster containing all genetic elements required to enable *E. coli* to recombinantly express the *P. aeruginosa* O6 O antigen, the *fmtO6* and O6wzy genes (i.e., the genes encoding SEQ ID NOs: 2 and 3, respectively) were cloned downstream of the *P. aeruginosa* O6 *rfb* cluster. A schematic representation of the cloning of the codon usage optimized *Pseudomonas aeruginosa* O6 O-antigen polymerase O6*wzy* into the cloned *Pseudomonas aeruginosa* O6 *rfb* cluster along with the O6 formyltransferase and the relative organization of the genes is depicted in Figure 5. It further was determined that the the *fmtO6* and O6*wzy* genes (i.e., the genes encoding SEQ ID NOs: 2 and 3, respectively) could be inserted into the *P. aeruginosa* O6 *rfb* cluster at multiple positions. Specifically, the *fmtO6* gene could be inserted in a clockwise orientation relative to the rfb cluster downstream of the rfb cluster or upstream of the rfb cluster under the control of a separate promotor. In addition, the *fmtO6* gene could be inserted in a counter-clockwise orientation relative to the rfb cluster upstream or downstream of the rfb cluster. The O6wzy gene could be inserted in a clockwise orientation relative to the rfb cluster upstream or downstream of the rfb cluster or upstream of the rfb cluster under the control of a separate promotor. All constructs described above were active in terms of *P. aeruginosa* O6 O antigen biosynthesis (data not shown).

### Example 4: Bacterial Strains with an Inserted Oligosaccharyl Transferase and an Inserted rfb, Completed rfbO6 Cluster Are Stable and Produce Bioconjugates

Example 1 demonstrates that bioconjugates can successfully be produced by a bacterial host strain that has been genetically modified by insertion of (i) a nucleic acid encoding an oligosaccharyl transferase and (ii) a nucleic acid encoding an *rfb* cluster. In this Example, experiments similar to those described in Example 1 were performed, using the *Pseudomonas* protein PcrV as a carrier protein.

Naturally, the primary amino acid sequence of PcrV (see, e.g., UniProt O30527) does not comprise an N-glycosylation consensus sequence ("glycosite"). Using the methods described in WO 2006/119987, recombinant variants of PcrV comprising one, two, three, four, or five glycosites were engineered. In particular, by manipulation of the nucleic acid sequence encoduing PcrV, PcrV variants were created that expressed one, two, three, four, or five of the optimized N-glycosylation consensuss sequence Asp(Glu)-X-Asn-Z-Ser(Thr), wherein X and Z are independently selected from any natural amino acid except Pro.

Modified *E. coli* host cells were generated by inserting the following directly into the host cell genome: (i) a nucleic acid encoding the *C. jejuni* oligosaccharyl transferase (PglB) and (ii) a nucleic acid encoding the *rfb* cluster from the *Pseudomonas aeruginosa* serotype O6 PAK strain. This *rfb* cluster encodes genes necessary for O-antigen synthesis of the *Pseudomonas aeruginosa* serogroup O6 antigen. The insertions were performed using the novel insertion method described in PCT/EP2013/071328 (see Section 5.2, above) or the pUT mini system (Biomedal Lifescience). The *E. coli* host cells further were modified by introduction of a plasmid that expresses PcrV comprising one to five glycosites, as described above. Thus, the modified *E. coli* host cells described in this example express (i) the *C. jejuni* oligosaccharyl transferase (PglB), by virtue of integration of a nucleic acid encoding the oligosaccharyl transferase into the host cell genome; (ii) genes of a *Pseudomonas aeruginosa rfb* cluster that produce the O6 antigen, by virtue of integration of a nucleic acid encoding the *rfb* cluster from *Pseudomonas aeruginosa* PAK strain into the host cell genome; and (iii) the modified PcrV carrier protein, by virtue of transforming the host cell with a plasmid comprising a modified nucleic acid encoding the carrier protein (where the nucleic acid has been modified so that it encodes one to five glycosites, as described above).

Additional modified *E. coli* host cells were generated to allow for comparison of the ability of the modified host cells comprising double integrations (integration of an oligosaccharyl transferase and integration of an *rfb* cluster) to produce bioconjugates (PcrV-O6) with bioconjugate production by host cells having (i) only a single integration of the oligosaccharyl transferase or the *rfb* cluster and the remaining components (carrier protein and oligosaccharyl transferase or *rfb* cluster) plasmid expressed by the host cell; or (ii) no integrated components, with all components (carrier protein and oligosaccharyl transferase and *rfb* cluster) plasmid expressed.

Three different *E. coli* strains were used and compared in the analysis: (i) "St7343," which comprises both *pglB* and the completed O6 *rfb* cluster inserted into the host cell genome (i.e., is a double integrated strain), and a plasmid encoding a PcrV carrier protein (with one, two, three, four, or five glycosites); (ii) "St7209," which comprises plasmid-expressed *pglB,* the O6 *rfb* cluster inserted into the host cell genome, and a plasmid encoding a PcrV carrier protein (with one, two, three, four, or five glycosites); and (iii) "St2182," which comprises plasmid-expressed *pglB,* plasmid-expressed O6 *rfb* cluster, and a plasmid encoding a PcrV carrier protein (with one, two, three, four, or five glycosites). Figure 6 depicts the characteristics of each strain (6A: St7343; 6B: St7209; 6C: St2182).

As shown in Figure 6, all strains expressing an oligosaccharyl transferase, carrier protein, and an *rfb* cluster produced bioconjugates. See the blots depicted between kDa markers 40-70 (around the kDa 55 marker), which correspond to PcrV-O6. Importantly, as shown in Example 1, this observation includes strains comprising double integration of an oligosaccharyl transferase and an *rfb* cluster. See, in particular, the results shown in Figure 6A. Thus, like Example 1, this Example demonstrates not only that stable host cells can be generated following double insertion of genes/gene clusters into the host cell genome, but that function of the genes is maintained. Specifically, function of the inserted oligosaccharyl transferase and inserted *rfb* cluster was preserved, resulting in the production of bioconjugates.

### Example 5: Production and purification of EPA-O6 bioconjugates

This example describes the production of bioconjugates comprising the *Pseudomonas aeruginosa* O6 antigen.

*E. coli* W3110 ΔwaaL Δwec Δrfb was transformed with plasmids comprising the *Pseudomonas aeruginosa* O6 *rfb* cluster, the oligosaccharyl transferase *pglB* from *C. jejuni,* the gene encoding the detoxified carrier protein EPA, and the QuiNAc biosynthesis/transferase genes *wbpVLM* (from a *Pseudomonas aeruginosa* O6 strain). Results of plasmid retention analysis are depicted in Figure 8. Medium (LB broth) supplemented with Tetracyclin, Spectinomycin, Kanamycin and Ampicillin was inoculated with host cells containing all four plasmids. The pre-culture was grown overnight at 37°C. The next day, medium (TB) supplemented with MgCl₂, Tetracyclin, Spectinomycin, Kanamycin and Ampicillin was inoculated by diluting the preculture to OD₆₀₀ 0.1. Cells were grown at 37°C until approximately OD₆₀₀ 0.8-1.0 was reached, then expression of *pglb, epa* and *wbpVLM* was induced by the addition of 1mM IPTG and 0.1% arabinose. Cells were harvested by centrifugation after over night induction.

EPA-06 bioconjugates were purified from periplasmic extracts of modified host cells using Metal-chelate affinity chromatography (IMAC), anion exchange chromatography (Source Q) and size exclusion chromatography (SEC). Elution fractions containing glycoconjugates were pooled and subsequently submitted to the next chromatography step. The final SEC eluates were characterized by SDS-PAGE followed by Coomassie Blue staining or Western blot using the antibodies indicated in Figure 7.

The EPA-06 bioconjugate was characterized using an array of analytical methods. The level of endotoxin was measured using the LAL assay (13EU/ml). Purity was determined by SDS-PAGE and capillary gel electrophoresis (CGE, 86% purity). The amount of protein was measured using the BCA assay (1.75mg/ml). The amount of polysaccharide was measured using the Anthrone assay (Dubois et al., 1956; 311.6ug/ml). The average size of the O6-Polymer was determined using a high resolution "degree-of-glycosylation" (DOG) SDS-PAGE (average of 7.9 repeating units per polymer). Determination of electric isoforms of the bioconjugate was done by isoelectric focusing (IEF). Finally, the identity of the bioconjugate was confirmed by Immunoblotting using antibodies directed against the protein (EPA) or the polysaccharide (O6).

### Example 6: Immunization Studies

This Example demonstrates that the *P. aeruginosa* O6-EPA bioconjugate is immunogenic.

Female, 6 week old BALB/c OlaHsd mice (in groups of 25) were immunized intramuscularly at days 0, 14 and 28 with 0.2 µg or 2 µg of 06-EPA conjugate (see Example 5) in a non adjuvanated or adjuvanated formulation (with an oil-in-water emulsion adjuvant). A control group of 10 mice was vaccinated with ajuvant (O/W) alone. Anti-O6 ELISA and opsonic titers were determined in individual sera collected at day 42 (14 post III) and on pooled Post -II and Post-III sera. Results are shown in Figure 9 and described in detail below.

Figure 9A depicts the anti-O6 ELISA response. Purified O6 LPS-O6 (PaO6a,6c) was coated at 8 µg/ml in phosphate buffered saline (PBS) on high-binding microtitre plates (Nunc Maxisorp), overnight at 4° C. The plates were blocked with PBS-BSA 1% for 30 min at RT with agitation. The mice antisera were prediluted 1/10 and then, further two fold dilutions were made in microplates and incubated at room temperature for 30 minutes with agitation. After washing, bound murine antibody was detected using Jackson ImmunoLaboratories Inc. peroxidase-conjugated AffiniPure Goat Anti-Mouse IgG (H+L) (ref: 115-035-003) diluted 1/5000 in PBS-tween 0.05%-BSA 0.2%. The detection antibodies were incubated for 30 minutes at room temperature with agitation. The color was developed using 4 mg OPD + 5 µl H2O2 per 10 ml pH 4.5 0.1M citrate buffer for 15 minutes in the dark at room temperature. The reaction was stopped with 50 µl HCl, and the optical density (OD) was read at 490 nm relative to 650 nm.

The level of anti-O6 antibodies present in the sera was expressed in mid-point titers. A GMT of individual sera was calculated for the 25 samples in each treatment group (10 for the control group).

An immune response was observed in mice after injection of the bioconjugate formulated with the adjuvant. No difference was observed between doses. Similar observations were made regarding the percentage of seroconversion. No or very weak responses were observed with the non adjuvanted formulation.

Figure 9B shows opsonic titer in HL60 cells from mice immunized with O6-EPA bioconjugate formulated with adjuvant or not.

The opsonophagocytosis assay (OPA) was performed in round-bottom microplates with 15 µl of HL-60 phagocytic cells (adjusted to 5 10e6 cells/ml), 15 µl of *P. aeruginosa* bacteria (grown on TSA agar plate), 15 µl of the test serum dilutions, and 15 µl of piglet complement. The inactivated test pooled sera were first diluted (1/16 or 1/50 final dilution) in HBSS-BSA 1% and added to a *P. aeruginosa* O6 strain (strain ID: HNCMB 170009, obtained from Hungarian National Collection of Medical Bacteria) diluted in order to count 200-250 CFU/well at the end of the test.

The HL-60 cells (adjusted to 5.10e6/ml) and the piglet complement (12.5% final) were then added in each well. A control with inactivated complement was included for each test sample.

The reaction mixture was incubated at 37°C for 90 minutes with agitation. After a 1/200 dilution, 50 µl of the volume was then transferred into a flat-bottom microplate. 50 µl of MH agar followed by PBS-0.9% agar was added. Automated colony counts were performed after an overnight incubation at 34°C.

The opsonophagocytic activity is expressed as the reciprocal of the serum dilution giving at least 50% killing.

The data demonstrate the functionality of the antibodies induced after injection with the adjuvanted group.

In conclusion, this example demonstrates that the *P. aeruginosa* O6-EPA bioconjugate is both immunogenic and functional (i.e., induces antibodies that kill *P. aeruginosa* O6 *in vivo*).

## Claims

1. A host cell comprising:
i. a nucleic acid that encodes a glycosyltransferase derived from an *rfb* cluster of *Pseudomonas;*
ii. a nucleic acid that encodes a formyltransferase enzyme, wherein said nucleic acid encodes a protein having about or at least 80%, 85%, 90%, 95%, 96%, 97%, 98%, or 99% identity or homology to SEQ ID NO:2, or wherein said nucleic acid encodes SEQ ID NO:2;
iii. a nucleic acid that encodes an oligosaccharyl transferase; and
iv. a nucleic acid that encodes a carrier protein comprising an N-glycosylation consensus sequence D/E - X - N - X- S/T, wherein X is any amino acid except proline.

2. The host cell of Claim 1, further comprising a nucleic acid that encodes a *wzy* polymerase, wherein said nucleic acid encodes a protein having about or at least 80%, 85%, 90%, 95%, 96%, 97%, 98%, or 99% identity or homology to SEQ ID NO:3, or wherein said nucleic acid encodes SEQ ID NO:3.

3. The host cell of any one of claims 1-2, wherein the glycosyltransferase is derived from an rfb cluster of *Pseudomonas aeruginosa* serotype 6.

4. The host cell of any one of claims 1-3, wherein said host cell is *E. coli.*

5. A method of producing a bioconjugate that comprises a *Pseudomonas* antigen linked to a carrier protein, said method comprising culturing the host cell of any one of claims 1-4 under conditions suitable for the production of proteins.

6. A bioconjugate produced by the method of claim 5, wherein said bioconjugate comprises a *Pseudomonas* antigen linked to a carrier protein.

7. A bioconjugate comprising a *Pseudomonas aeruginosa* serotype O6 antigen conjugated to a carrier protein via the N residue of at least one N-glycosylation consensus sequence D/E-X-N-X-S/T, wherein X is any amino acid except proline wherein the O6 antigen is formylated.

8. The bioconjugate of claim 7, wherein the O6 antigen comprises a formyl group attached to at least 1 GalNFmA residue.

9. The bioconjugate of claim 8, wherein at least 10, 20, 30, 40, 50, 60, 70, 80, 90, 95 or 100% of O antigen repeat units are formylated.

10. The bioconjugate of any one of claims 8-9 for use in the treatment or prevention of a disease caused by a *Pseudomonas* infection in a human subject.

11. The bioconjugate of any one of claims 8-9 for use in the manufacture of a medicament for the treatment or prevention of a disease caused by *Pseudomonas* in a human subject.

## Patentansprüche

1. Wirtszelle, umfassend:
i. eine Nukleinsäure, die eine von einem rfb-Cluster von *Pseudomonas* abgeleitete Glykosyltransferase kodiert;
ii. eine Nukleinsäure, die ein Formyltransferase-Enzym kodiert, wobei die Nukleinsäure ein Protein mit etwa oder mindestens 80%, 85%, 90%, 95%, 96%, 97%, 98% oder 99% Identität mit oder Homologie zu SEQ ID NO:2 kodiert, oder wobei die Nukleinsäure SEQ ID NO:2 kodiert;
iii. eine Nukleinsäure, die eine Oligosaccharyltransferase kodiert; und
iv. eine Nukleinsäure, die ein Trägerprotein kodiert, welches eine N-Glykosylierungskonsensussequenz D/E-X-N-X-S/T umfasst, wobei X irgendeine Aminosäure außer Prolin ist.

2. Wirtszelle gemäß Anspruch 1, weiter umfassend eine Nukleinsäure, die eine wzy-Polymerase kodiert, wobei die Nukleinsäure ein Protein mit etwa oder mindestens 80%, 85%, 90%, 95%, 96%, 97%, 98% oder 99% Identität mit oder Homologie zu SEQ ID NO:3 kodiert, oder wobei die Nukleinsäure SEQ ID NO:3 kodiert.

3. Wirtszelle gemäß irgendeinem der Ansprüche 1-2, wobei die Glykosyltransferase von einem rfb-Cluster von *Pseudomonas aeruginosa* Serotyp 6 abgeleitet ist.

4. Wirtszelle gemäß irgendeinem der Ansprüche 1-3, wobei die Wirtszelle *E. coli* ist.

5. Verfahren zur Herstellung eines Biokonjugats, das ein mit einem Trägerprotein verbundenes *Pseudomonas-Antigen* umfasst, wobei das Verfahren das Kultivieren der Wirtszelle gemäß irgendeinem der Ansprüche 1-4 unter Bedingungen umfasst, die zur Herstellung von Proteinen geeignet sind.

6. Biokonjugat, das durch das Verfahren gemäß Anspruch 5 hergestellt ist, wobei das Biokonjugat ein mit einem Trägerprotein verbundenes *Pseudomonas-Antigen* umfasst.

7. Biokonjugat, das ein *Pseudomonas aeruginosa* Serotyp O6-Antigen umfasst, das mittels des N-Rests mindestens einer N-Glykosylierungskonsensussequenz D/E-X-N-X-S/T an ein Trägerprotein konjugiert ist, wobei X irgendeine Aminosäure außer Prolin ist, wobei das O6-Antigen formyliert ist.

8. Biokonjugat gemäß Anspruch 7, wobei das O6-Antigen eine Formylgruppe umfasst, die an mindestens 1 GalNFmA-Rest angehängt ist.

9. Biokonjugat gemäß Anspruch 8, wobei mindestens 10, 20, 30, 40, 50, 60, 70, 80, 90, 95 oder 100% der O-Antigen-Wiederholungseinheiten (O antigen repeat units) formyliert sind.

10. Biokonjugat gemäß irgendeinem der Ansprüche 8-9 zur Verwendung bei der Behandlung oder Prävention einer Krankheit, die durch eine Pseudomonas-Infektion verursacht ist, in einem menschlichen Subjekt.

11. Biokonjugat gemäß irgendeinem der Ansprüche 8-9 zur Verwendung bei der Herstellung eines Medikaments zur Behandlung oder Prävention einer Krankheit, die durch *Pseudomonas* verursacht ist, in einem menschlichen Subjekt.

## Revendications

1. Cellule hôte comprenant :
i. un acide nucléique qui code pour une glycosyltransférase dérivée d'un groupe *rfb* de *Pseudomonas ;*
ii. un acide nucléique qui code pour une enzyme formyltransférase, dans laquelle ledit acide nucléique code pour une protéine présentant environ ou au moins 80 %, 85 %, 90 %, 95 %, 96 %, 96 %, 97 %, 98 % ou 99 % d'identité ou d'homologies avec SEQ ID NO : 2, ou dans lequel ledit acide nucléique code pour SEQ ID NO : 2 ;
iii. un acide nucléique qui code pour une oligosaccharyltransférase ; et
iv. un acide nucléique qui code pour une protéine porteuse comprenant une séquence consensus de N-glycosylation D/E-X-N-S/T, dans laquelle X est un acide aminé quelconque à l'exception de la proline.

2. Cellule hôte selon la revendication 1, comprenant en outre un acide nucléique qui code pour une polymérase *wzy,* dans laquelle ledit acide nucléique code pour une protéine présentant environ ou au moins 80 %, 85 %, 90 %, 95 %, 96 %, 97 %, 98 %, ou 99 % d'identité ou d'homologie avec SEQ ID NO : 3, ou dans laquelle ledit acide nucléique code pour SEQ ID NO : 3.

3. Cellule hôte selon l'une quelconque des revendications 1 et 2, dans laquelle la glycosyltransférase est dérivée d'un groupe rfb de *Pseudomonas aeruginosa* de sérotype 6.

4. Cellule hôte selon l'une quelconque des revendications 1 à 3, dans laquelle ladite cellule hôte est *E. coli.*

5. Procédé de production d'un bioconjugué qui comprend un antigène de *Pseudomonas* lié à une protéine porteuse, ledit procédé comprenant la culture de la cellule hôte de l'une quelconque des revendications 1 à 4 dans des conditions appropriées pour la production de protéines.

6. Bioconjugué produit par le procédé de la revendication 5, dans lequel ledit bioconjugué comprend un antigène de *Pseudomonas* lié à une protéine porteuse.

7. Bioconjugué comprenant un antigène de *Pseudomonas aeruginosa* de sérotype O6 conjugué à une protéine porteuse via le résidu N d'au moins une séquence consensus de N-glycosylation D/E-X-N-X_S/T, dans lequel X est un acide aminé à l'exception de la proline dans lequel l'antigène O6 est formylé.

8. Bioconjugué selon la revendication 7, dans lequel l'antigène O6 comprend un groupe formyle attaché à au moins 1 résidu GaINFmA.

9. Bioconjugué selon la revendication 8, dans lequel au moins 10, 20, 30, 40, 50, 60, 70, 80, 90, 95 ou 100 % des unités répétées d'antigène O sont formylées.

10. Bioconjugué selon l'une quelconque des revendications 8 à 9, destiné à être utilisé dans le traitement ou la prévention d'une maladie causée par une infection à *Pseudomonas* chez un sujet humain.

11. Bioconjugué selon l'une quelconque des revendications 8 à 9, destiné à être utilisé dans la fabrication d'un médicament destiné au traitement ou à la prévention d'une maladie causée par *Pseudomonas* chez un sujet humain.
